# EUROPEAN PATENT SPECIFICATION

(11) **EP 4 567 716 B1**
(45) Date of publication and mention of the grant of the patent: **13.05.2026**
(21) Application number: 24216713.8
(22) Date of filing: 02.12.2024
(51) Int. Cl.: G06T 5/70, G16H 30/40, G06T 7/00, G06T 5/60, A61B 6/00, G06N 3/045, G06N 3/047

(54) **GENERATING SYNTHETIC REPRESENTATIONS**
ERZEUGUNG SYNTHETISCHER DARSTELLUNGEN
GÉNÉRATION DE REPRÉSENTATIONS SYNTHÉTIQUES

(30) Priority: 06.12.2023 EP 23214619; 10.06.2024 EP 24181125
(43) Date of publication of application: 11.06.2025
(73) Proprietor: Bayer Aktiengesellschaft, 51373 Leverkusen (DE)
(72) Inventor: Mohammadi, Seyed Sadegh, 50968 Köln (DE); Montalt Tordera, Javier, 46014 Valencia (ES); Prokop, Jakub, 02-326 Warschau (PL)
(74) Representative: BIP Patents

(56) References cited:
- WO-A1-2022/171597
- WO-A1-2022/171845
- WO-A1-2023/073165
- US-A1- 2021 241 458

## Description

### FIELD OF THE DISCLOSURE

Systems, methods, and computer programs disclosed herein relate to generating synthetic contrast-enhanced radiologic images.

### BACKGROUND

WO2019/074938A1 and WO2022184297A1 describe methods for generating a synthetic contrast-enhanced radiological image showing an examination area of an examination object after application of a standard amount of a contrast agent, although only a smaller amount of contrast agent than the standard amount was applied. The standard amount is the amount recommended by the manufacturer and/or distributor of the contrast agent and/or the amount approved by a regulatory authority and/or the amount listed in a package insert for the contrast agent. The methods described in WO2019/074938A1 and WO2022184297A can therefore be used to reduce the amount of contrast agent.

The methods described in WO2019/074938A1 and WO2022184297A1 use machine learning models that have been trained based on training data. For each examination object of a plurality of examination objects, the training data comprises a native radiological image and a radiological image after application of an amount of the contrast agent that is smaller than the standard amount as input data and a radiological image after application of the standard amount of the contrast agent as target data. The training procedure cannot be carried out if, for example, the target data is not available. In order to generate a synthetic radiological image that represents an examination area of an examination object after application of a larger than standard amount of contrast agent, corresponding target data would have to be available, i.e. a larger than standard amount of contrast agent would have to be administered to examination objects.

### SUMMARY

These problems are addressed by the subject matter of the independent claims of the present disclosure. Exemplary embodiments are defined in the dependent claims, the description, and the drawings.

In a first aspect, the present disclosure relates to a computer-implemented method comprising:
- providing a generative machine learning model,
- providing training data, wherein the training data comprises, for each reference object of a plurality of reference objects, a first reference representation representing an examination area of the reference object without contrast agent, and a second reference representation representing the examination area of the reference object after application of an amount of a contrast agent,
- training the generative machine learning model, wherein training the generative machine learning model comprises, for each reference object of the plurality of reference objects:
   - causing the generative machine learning model to generate a first synthetic representation of the examination area of the reference object based on the first reference representation and/or the second reference representation,
   - generating a second synthetic representation of the examination area of the reference object, wherein generating the second synthetic representation comprises: subtracting the first reference representation from the first synthetic representation,
   - generating a third synthetic representation of the examination area of the reference object, wherein generating the third synthetic representation comprises: reducing the contrast of the second synthetic representation,
   - generating a fourth synthetic representation of the examination area of the reference object, wherein generating the fourth synthetic representation comprises: subtracting the third synthetic representation from the first synthetic representation or adding the third synthetic representation to the first reference representation,
   - determining deviations between the fourth synthetic representation and the second reference representation,
   - reducing the deviations by modifying parameters of the generative machine learning model,
- storing the trained generative machine learning model and/or transmitting the trained generative machine learning model to another computer system and/or using the trained generative machine learning model to generate a synthetic representation of the examination area of an examination object.

**In** another aspect, the present disclosure relates to a computer-implemented method comprising:
- providing a trained generative machine learning model,
   - wherein the trained generative machine learning model was trained on training data,
   - wherein the training data comprised, for each reference object of a plurality of reference objects, a first reference representation representing an examination area of the reference object without contrast agent, and a second reference representation representing the examination area of the reference object after application of an amount of a contrast agent,
   - wherein training the generative machine learning model comprised, for each reference object of the plurality of reference objects:
      ∘ causing the generative machine learning model to generate a first synthetic representation of the examination area of the reference object based on the first reference representation and/or the second reference representation,
      ∘ generating a second synthetic representation of the examination area of the reference object, wherein generating the second synthetic representation comprises: subtracting the first reference representation from the first synthetic representation,
      ∘ generating a third synthetic representation of the examination area of the reference object, wherein generating the third synthetic representation comprises: reducing the contrast of the second synthetic representation,
      ∘ generating a fourth synthetic representation of the examination area of the reference object, wherein generating the fourth synthetic representation comprises: subtracting the third synthetic representation from the first synthetic representation or adding the third synthetic representation to the first reference representation,
      ∘ determining deviations between the fourth synthetic representation and the second reference representation,
      ∘ reducing the deviations by modifying parameters of the generative machine learning model,
- providing a first representation and/or a second representation of the examination area of an examination object, wherein the first representation represents the examination area of the examination object without contrast agent, and the second representation represents the examination area of the examination object after application of the amount of the contrast agent,
- causing the trained generative machine learning model to generate a synthetic representation of the examination area of the examination object based on the first representation and/or the second representation,
- outputting the synthetic representation of the examination area of the examination object and/or storing the synthetic representation of the examination area of the examination object in a data storage and/or transmitting the synthetic representation of the examination area of the examination object to a separate computer system.

In another aspect, the present disclosure provides a computer system comprising:
a processing unit; and
a memory storing a computer program configured to perform, when executed by the processing unit, an operation, the operation comprising:
   - providing a trained generative machine learning model,
      - wherein the trained generative machine learning model was trained on training data,
      - wherein the training data comprised, for each reference object of a plurality of reference objects, a first reference representation representing an examination area of the reference object without contrast agent, and a second reference representation representing the examination area of the reference object after application of an amount of a contrast agent,
      - wherein training the generative machine learning model comprised, for each reference object of the plurality of reference objects:
         ∘ causing the generative machine learning model to generate a first synthetic representation of the examination area of the reference object based on the first reference representation and/or the second reference representation,
         ∘ generating a second synthetic representation of the examination area of the reference object, wherein generating the second synthetic representation comprises: subtracting the first reference representation from the first synthetic representation,
         ∘ generating a third synthetic representation of the examination area of the reference object, wherein generating the third synthetic representation comprises: reducing the contrast of the second synthetic representation,
         ∘ generating a fourth synthetic representation of the examination area of the reference object, wherein generating the fourth synthetic representation comprises: subtracting the third synthetic representation from the first synthetic representation or adding the third synthetic representation to the first reference representation,
         ∘ determining deviations between the fourth synthetic representation and the second reference representation,
         ∘ reducing the deviations by modifying parameters of the generative machine learning model,
   - providing a first representation and/or a second representation of the examination area of an examination object, wherein the first representation represents the examination area of the examination object without contrast agent, and the second representation represents the examination area of the examination object after application of the amount of the contrast agent,
   - causing the trained generative machine learning model to generate a synthetic representation of the examination area of the examination object based on the first representation and/or the second representation,
   - outputting the synthetic representation of the examination area of the examination object and/or storing the synthetic representation of the examination area of the examination object in a data storage and/or transmitting the synthetic representation of the examination area of the examination object to a separate computer system.

In another aspect, the present disclosure provides a non-transitory computer readable storage medium having stored thereon software instructions that, when executed by a processing unit of a computer system, cause the computer system to perform the following steps:
- providing a trained generative machine learning model,
   - wherein the trained generative machine learning model was trained on training data,
   - wherein the training data comprised, for each reference object of a plurality of reference objects, a first reference representation representing an examination area of the reference object without contrast agent, and a second reference representation representing the examination area of the reference object after application of an amount of a contrast agent,
   - wherein training the generative machine learning model comprised, for each reference object of the plurality of reference objects:
      ∘ causing the generative machine learning model to generate a first synthetic representation of the examination area of the reference object based on the first reference representation and/or the second reference representation,
      ∘ generating a second synthetic representation of the examination area of the reference object, wherein generating the second synthetic representation comprises: subtracting the first reference representation from the first synthetic representation,
      ∘ generating a third synthetic representation of the examination area of the reference object, wherein generating the third synthetic representation comprises: reducing the contrast of the second synthetic representation,
      ∘ generating a fourth synthetic representation of the examination area of the reference object, wherein generating the fourth synthetic representation comprises: subtracting the third synthetic representation from the first synthetic representation or adding the third synthetic representation to the first reference representation,
      ∘ determining deviations between the fourth synthetic representation and the second reference representation,
      ∘ reducing the deviations by modifying parameters of the generative machine learning model,
- providing a first representation and/or a second representation of the examination area of an examination object, wherein the first representation represents the examination area of the examination object without contrast, and the second representation represents the examination area of the examination object after application of the amount of the contrast agent,
- causing the trained generative machine learning model to generate a synthetic representation of the examination area of the examination object based on the first representation and/or the second representation,
- outputting the synthetic representation of the examination area of the examination object and/or storing the synthetic representation of the examination area of the examination object in a data storage and/or transmitting the synthetic representation of the examination area of the examination object to a separate computer system.

In another aspect, the present disclosure relates to a use of a contrast agent in an examination of an examination area of an examination object, the examination comprising:
- providing a trained generative machine learning model,
   - wherein the trained generative machine learning model was trained on training data,
   - wherein the training data comprised, for each reference object of a plurality of reference objects, a first reference representation representing an examination area of the reference object without contrast agent, and a second reference representation representing the examination area of the reference object after application of an amount of the contrast agent,
   - wherein training the generative machine learning model comprised, for each reference object of the plurality of reference objects:
      ∘ causing the generative machine learning model to generate a first synthetic representation of the examination area of the reference object based on the first reference representation and/or the second reference representation,
      ∘ generating a second synthetic representation of the examination area of the reference object, wherein generating the second synthetic representation comprises: subtracting the first reference representation from the first synthetic representation,
      ∘ generating a third synthetic representation of the examination area of the reference object, wherein generating the third synthetic representation comprises: reducing the contrast of the second synthetic representation,
      ∘ generating a fourth synthetic representation of the examination area of the reference object, wherein generating the fourth synthetic representation comprises: subtracting the third synthetic representation from the first synthetic representation or adding the third synthetic representation to the first reference representation,
      ∘ determining deviations between the fourth synthetic representation and the second reference representation,
      ∘ reducing the deviations by modifying parameters of the generative machine learning model,
- generating a first representation and/or a second representation of the examination area of the examination object, wherein the first representation represents the examination area of the examination object without contrast agent, and the second representation represents the examination area of the examination object after application of the amount of the contrast agent,
- causing the trained generative machine learning model to generate a synthetic representation of the examination area of the examination object based on the first representation and/or the second representation,
- outputting the synthetic representation of the examination area of the examination object and/or storing the synthetic representation of the examination area of the examination object in a data storage and/or transmitting the synthetic representation of the examination area of the examination object to a separate computer system.

In another aspect, the present disclosure relates to a contrast agent for use in an examination of an examination area of an examination object, the examination comprising:
- providing a trained generative machine learning model,
   - wherein the trained generative machine learning model was trained on training data,
   - wherein the training data comprised, for each reference object of a plurality of reference objects, a first reference representation representing an examination area of the reference object without contrast agent, and a second reference representation representing the examination area of the reference object after application of an amount of the contrast agent,
   - wherein training the generative machine learning model comprised, for each reference object of the plurality of reference objects:
      ∘ causing the generative machine learning model to generate a first synthetic representation of the examination area of the reference object based on the first reference representation and/or the second reference representation,
      ∘ generating a second synthetic representation of the examination area of the reference object, wherein generating the second synthetic representation comprises: subtracting the first reference representation from the first synthetic representation,
      ∘ generating a third synthetic representation of the examination area of the reference object, wherein generating the third synthetic representation comprises: reducing the contrast of the second synthetic representation,
      ∘ generating a fourth synthetic representation of the examination area of the reference object, wherein generating the fourth synthetic representation comprises: subtracting the third synthetic representation from the first synthetic representation or adding the third synthetic representation to the first reference representation,
      ∘ determining deviations between the fourth synthetic representation and the second reference representation,
      ∘ reducing the deviations by modifying parameters of the generative machine learning model,
- generating a first representation and/or a second representation of the examination area of the examination object, wherein the first representation represents the examination area of the examination object without contrast agent, and the second representation represents the examination area of the examination object after application of the amount of the contrast agent,
- causing the trained generative machine learning model to generate a synthetic representation of the examination area of the examination object based on the first representation and/or the second representation,
- outputting the synthetic representation of the examination area of the examination object and/or storing the synthetic representation of the examination area of the examination object in a data storage and/or transmitting the synthetic representation of the examination area of the examination object to a separate computer system.

In another aspect, the present disclosure provides a kit comprising a contrast agent and computer-readable program code that, when executed by a processing unit of a computer system, cause the computer system to execute the following steps:
- providing a trained generative machine learning model,
   - wherein the trained generative machine learning model was trained on training data,
   - wherein the training data comprised, for each reference object of a plurality of reference objects, a first reference representation representing an examination area of the reference object without contrast agent, and a second reference representation representing the examination area of the reference object after application of an amount of the contrast agent,
   - wherein training the generative machine learning model comprised, for each reference object of the plurality of reference objects:
      ∘ causing the generative machine learning model to generate a first synthetic representation of the examination area of the reference object based on the first reference representation and/or the second reference representation,
      ∘ generating a second synthetic representation of the examination area of the reference object, wherein generating the second synthetic representation comprises: subtracting the first reference representation from the first synthetic representation,
      ∘ generating a third synthetic representation of the examination area of the reference object, wherein generating the third synthetic representation comprises: reducing the contrast of the second synthetic representation,
      ∘ generating a fourth synthetic representation of the examination area of the reference object, wherein generating the fourth synthetic representation comprises: subtracting the third synthetic representation from the first synthetic representation or adding the third synthetic representation to the first reference representation,
      ∘ determining deviations between the fourth synthetic representation and the second reference representation,
      ∘ reducing the deviations by modifying parameters of the generative machine learning model,
- generating a first representation and/or a second representation of the examination area of an examination object, wherein the first representation represents the examination area of the examination object without contrast agent, and the second representation represents the examination area of the examination object after application of the amount of the contrast agent,
- causing the trained generative machine learning model to generate a synthetic representation of the examination area of the examination object based on the first representation and/or the second representation,
- outputting the synthetic representation of the examination area of the examination object and/or storing the synthetic representation of the examination area of the examination object in a data storage and/or transmitting the synthetic representation of the examination area of the examination object to a separate computer system.

### BRIEF DESCRIPTION OF THE DRAWINGS

Fig. 1 shows schematically an example of training a generative machine learning model.
Fig. 2 exemplarily and schematically illustrates the generation of a synthetic representation of the examination area of an examination object.
Fig. 3 shows an embodiment of the method of training the machine learning model in the form of a flow chart.
Fig. 4 shows an embodiment of the method of generating a synthetic representation in the form of a flow chart.
Fig. 5 illustrates a computer system according to some example implementations of the present disclosure in more detail.

### DETAILED DESCRIPTION

Various example embodiments will be more particularly elucidated below without distinguishing between the aspects of the disclosure (computer-implemented methods, computer system, computer-readable storage medium, use, contrast agent for use, kit). On the contrary, the following elucidations are intended to apply analogously to all the aspects of the disclosure, irrespective of in which context (computer-implemented methods computer system, computer-readable storage medium, use, contrast agent for use, kit) they occur.

If steps are stated in an order in the present description or in the claims, this does not necessarily mean that the disclosure is restricted to the stated order. On the contrary, it is conceivable that the steps can also be executed in a different order or else in parallel to one another, unless, for example one step builds upon another step, this requiring that the building step be executed subsequently (this being, however, clear in the individual case). The stated orders may thus be exemplary embodiments of the present disclosure.

As used herein, the articles "a" and "an" are intended to include one or more items and may be used interchangeably with "one or more" and "at least one." As used in the specification and the claims, the singular form of "a", "an", and "the" include plural referents, unless the context clearly dictates otherwise. Where only one item is intended, the term "one" or similar language is used. Also, as used herein, the terms "has", "have", "having", or the like are intended to be open-ended terms. Further, the phrase "based on" is intended to mean "based at least partially on" unless explicitly stated otherwise. Further, the phrase "based on" may mean "in response to" and be indicative of a condition for automatically triggering a specified operation of an electronic device (e.g., a controller, a processor, a computing device, etc.) as appropriately referred to herein.

Some implementations of the present disclosure will be described more fully hereinafter with reference to the accompanying drawings, in which some, but not all implementations of the disclosure are shown.

Indeed, various implementations of the disclosure may be embodied in many different forms and should not be construed as limited to the implementations set forth herein; rather, these example implementations are provided so that this disclosure will be thorough and complete, and will fully convey the scope of the disclosure to those skilled in the art.

The terms used in this disclosure have the meaning that these terms have in the prior art, in particular in the prior art cited in this disclosure, unless otherwise indicated.

The present disclosure provides means for generating a synthetic representation of an examination area of an examination object.

In an embodiment of the present disclosure, the "examination object" is a living being.

In an embodiment of the present disclosure, the "examination object" is a mammal.

In an embodiment of the present disclosure, the "examination object" is a human.

The "examination area" is a part of the examination object, for example an organ or part of an organ or a plurality of organs or another part of the examination object.

For example, the examination area may be a liver, kidney, heart, lung, brain, stomach, bladder, prostate, intestine, breast, thyroid, pancreas, uterus or a part of said parts or another part of the body of a mammal (for example a human).

In an embodiment, the examination area includes a liver or part of a liver or the examination area is a liver or part of a liver of a mammal, e.g. a human.

In a further embodiment, the examination area includes a brain or part of a brain or the examination area is a brain or part of a brain of a mammal, e.g. a human.

In a further embodiment, the examination area includes a heart or part of a heart or the examination area is a heart or part of a heart of a mammal, e.g. a human.

In a further embodiment, the examination area includes a thorax or part of a thorax or the examination area is a thorax or part of a thorax of a mammal, e.g. a human.

In a further embodiment, the examination area includes a stomach or part of a stomach or the examination area is a stomach or part of a stomach of a mammal, e.g. a human.

In a further embodiment, the examination area includes a pancreas or part of a pancreas or the examination area is a pancreas or part of a pancreas of a mammal, e.g. a human.

In a further embodiment, the examination area includes a kidney or part of a kidney or the examination area is a kidney or part of a kidney of a mammal, e.g. a human.

In a further embodiment, the examination area includes one or both lungs or part of a lung of a mammal, e.g. a human.

In a further embodiment, the examination area includes a breast or part of a breast or the examination area is a breast or part of a breast of a female mammal, e.g. a female human.

In a further embodiment, the examination area includes a prostate or part of a prostate or the examination area is a prostate or part of a prostate of a male mammal, e.g. a male human.

The term "synthetic" means that the synthetic representation is not the (direct) result of a physical measurement on a real object under examination, but that the synthetic representation has been generated by a generative machine learning model. A synonym for the term "synthetic" is the term "artificial". A synthetic representation may however be based on one or more measured representations, i.e., the generative machine learning model may be able to generate the synthetic representation based on one or more measured representations (and/or other/further data).

The synthetic representation is generated based on a native representation of the examination area of the examination object and/or a contrast-enhanced representation of the examination area of the examination object. The generation of the synthetic representation may be based on further data.

The native representation represents the examination area of the examination object without contrast agent. The native representation is also referred to as the first representation in this disclosure.

The contrast-enhanced representation represents the examination area of the examination object after administration of a first amount of a contrast agent. The contrast-enhanced representation is also referred to as the second representation in this disclosure.

"Contrast agents" are substances or mixtures of substances that improve the depiction of structures and functions of the body in radiological examinations.

In computed tomography, iodine-containing solutions are usually used as contrast agents. In magnetic resonance imaging (MRI), superparamagnetic substances (for example iron oxide nanoparticles, superparamagnetic iron-platinum particles (SIPPs)) or paramagnetic substances (for example gadolinium chelates, manganese chelates, hafnium chelates) are usually used as contrast agents. In the case of sonography, liquids containing gas-filled microbubbles are usually administered intravenously. In positron emission tomography (PET) radiotracers are used as contrast agents. Contrast in PET images is caused by the differential uptake of the radiotracer in different tissues or organs. A radiotracer is a radioactive substance that is injected into the examination object. The radiotracer emits positrons. When a positron collides with an electron within the examination area of the examination object, both particles are annihilated, producing two gamma rays that are emitted in opposite directions. These gamma rays are then detected by a PET scanner, allowing the creation of detailed images of the body's internal functioning.

Examples of contrast agents can be found in the literature (see for example A.S.L. Jascinth et al.: Contrast Agents in computed tomography: A Review, Journal of Applied Dental and Medical Sciences, 2016, vol. 2, issue 2, 143-149; H. Lusic et al.: X-ray-Computed Tomography Contrast Agents, Chem. Rev. 2013, 113, 3, 1641-1666; https://www.radiology.wisc.edu/wp-content/uploads/2017/10/contrast-agents-tutorial.pdf, M.R. Nouh et al.: Radiographic and magnetic resonances contrast agents: Essentials and tips for safe practices, World J Radiol. 2017 Sep. 28; 9(9): 339-349; L.C. Abonyi et al.: Intravascular Contrast Media in Radiography: Historical Development & Review of Risk Factors for Adverse Reactions, South American Journal of Clinical Research, 2016, vol. 3, issue 1, 1-10; ACR Manual on Contrast Media, 2020, ISBN: 978-1-55903-012-0; A. Ignee et al.: Ultrasound contrast agents, Endosc Ultrasound. 2016 Nov-Dec; 5(6): 355-362; J. Trotter et al.: Positron Emission Tomography (PET)/Computed Tomography (CT) Imaging in Radiation Therapy Treatment Planning: A Review of PET Imaging Tracers and Methods to Incorporate PET/CT, Advances in Radiation Oncology (2023) 8, 101212).

In an embodiment of the present disclosure, the contrast agent is a hepatobiliary contrast agent.

A hepatobiliary contrast agent has the characteristic features of being specifically taken up by liver cells (hepatocytes), accumulating in the functional tissue (parenchyma) and enhancing contrast in healthy liver tissue. An example of a hepatobiliary contrast agent is the disodium salt of gadoxetic acid (Gd-EOB-DTPA disodium), which is described in US Patent No. 6 039 931A and is commercially available under the trade names Primovist^{®} and Eovist^{®}. Further hepatobiliary contrast agents are described *inter alia* in WO2022/194777.

In an embodiment of the present disclosure, the contrast agent is an agent that includes gadolinium(III) 2-[4,7,10-tris(carboxymethyl)-1,4,7,10-tetrazacyclododec-1-yl]acetic acid (also referred to as gadolinium-DOTA or gadoteric acid).

In a further embodiment, the contrast agent is an agent that includes gadolinium(III) ethoxybenzyldiethylenetriaminepentaacetic acid (Gd-EOB-DTPA); preferably, the contrast agent includes the disodium salt of gadolinium(III) ethoxybenzyldiethylenetriaminepentaacetic acid (also referred to as gadoxetic acid).

In an embodiment of the present disclosure, the contrast agent is an agent that includes gadolinium(III) 2-[3,9-bis[1-carboxylato-4-(2,3-dihydroxypropylamino)-4-oxobutyl]-3,6,9,15-tetrazabicyclo[9.3.1]pentadeca-1(15),11,13-trien-6-yl]-5-(2,3-dihydroxypropylamino)-5-oxopentanoate (also referred to as gadopiclenol) (see for example WO2007/042504 and WO2020/030618 and/or WO2022/013454).

In an embodiment of the present disclosure, the contrast agent is an agent that includes dihydrogen [(±)-4-carboxy-5,8,11-tris(carboxymethyl)-1-phenyl-2-oxa-5,8,11-triazatridecan-13-oato(5-)]gadolinate(2-) (also referred to as gadobenic acid).

In an embodiment of the present disclosure, the contrast agent is an agent that includes tetragadolinium [4,10-bis(carboxylatomethyl)-7-{3,6,12,15-tetraoxo-16-[4,7,10-tris-(carboxylatomethyl)-1,4,7,10-tetraazacyclododecan-1-yl]-9,9-bis({[({2-[4,7,10-tris-(carboxylatomethyl)-1,4,7,10-tetraazacyclododecan-1-yl]propanoyl}amino)acetyl]-amino}methyl)-4,7,11,14-tetraazahepta-decan-2-yl}-1,4,7,10-tetraazacyclododecan-1-yl]acetate (also referred to as gadoquatrane) (see for example J. Lohrke et al.: Preclinical Profile of Gadoquatrane: A Novel Tetrameric, Macrocyclic High Relaxivity Gadolinium-Based Contrast Agent. Invest Radiol., 2022, 1, 57(10): 629-638; WO2016193190).

In an embodiment of the present disclosure, the contrast agent is an agent that includes a Gd³⁺ complex of a compound of the formula (I) 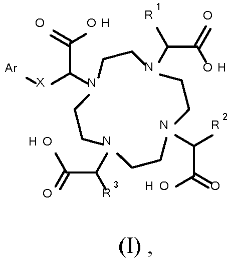 where
- Ar: is a group selected from

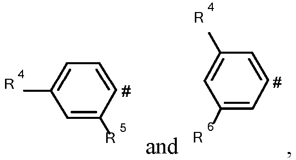

where # is the linkage to X,
X is a group selected from
   CH₂, (CH₂)₂, (CH₂)₃, (CH₂)₄ and *-(CH₂)₂-O-CH₂-^{#},
where * is the linkage to Ar and ^{#} is the linkage to the acetic acid residue,
R¹, R² and R³ are each independently a hydrogen atom or a group selected from C₁-C₃ alkyl, -CH₂OH, -(CH₂)₂OH and -CH₂OCH₃,
R⁴ is a group selected from C₂-C₄ alkoxy, (H₃C-CH₂)-O-(CH₂)₂-O-, (H₃C-CH₂)-O-(CH₂)₂-O-(CH₂)₂-O- and (H₃C-CH₂)-O-(CH₂)₂-O-(CH₂)₂-O-(CH₂)₂-O-,
R⁵ is a hydrogen atom, and
   - R⁶: is a hydrogen atom,
or a stereoisomer, tautomer, hydrate, solvate or salt thereof, or a mixture thereof.

In one embodiment of the present disclosure, the contrast agent is an agent that includes a Gd³⁺ complex of a compound of the formula (II) 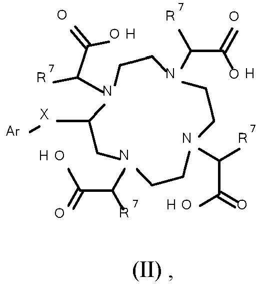 where Ar is a group selected from 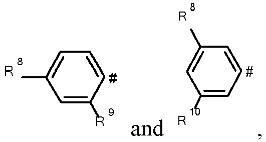
where # is the linkage to X,
X is a group selected from CH₂, (CH₂)₂, (CH₂)₃, (CH₂)₄ and *-(CH₂)₂-O-CH₂-^{#}, where * is the linkage to Ar and ^{#} is the linkage to the acetic acid residue,
R⁷ is a hydrogen atom or a group selected from C₁-C₃ alkyl, -CH₂OH,
   -(CH₂)₂OH and -CH₂OCH₃;
R⁸ is a group selected from
C₂-C₄ alkoxy, (H₃C-CH₂O)-(CH₂)₂-O-, (H₃C-CH₂O)-(CH₂)₂-O-(CH₂)₂-O- and
(H₃C-CH₂O)-(CH₂)₂-O-(CH₂)₂-O-(CH₂)₂-O-;
R⁹ and R¹⁰ independently represent a hydrogen atom;
or a stereoisomer, tautomer, hydrate, solvate or salt thereof, or a mixture thereof.

The term "C₁-C₃ alkyl" denotes a linear or branched, saturated monovalent hydrocarbon group having 1, 2 or 3 carbon atoms, for example methyl, ethyl, n-propyl or isopropyl. The term "C₂-C₄ alkyl" denotes a linear or branched, saturated monovalent hydrocarbon group having 2, 3 or 4 carbon atoms.

The term "C₂-C₄" refers to a linear or branched, saturated monovalent group of the formula (C₂-C₄ alkyl)-O-, in which the term "C₂-C₄ alkyl" is as defined above, for example a methoxy, ethoxy, n-propoxy or isopropoxy group.

In an embodiment of the present disclosure, the contrast agent is an agent that includes gadolinium 2,2',2"-(10-{1-carboxy-2-[2-(4-ethoxyphenyl)ethoxy]ethyl}-1,4,7,10-tetraazacyclododecane-1,4,7-triyl)triacetate (see for example WO2022/194777, example 1).

In an embodiment of the present disclosure, the contrast agent is an agent that includes gadolinium 2,2',2"-{10-[1-carboxy-2-{4-[2-(2-ethoxyethoxy)ethoxy]phenyl}ethyl]-1,4,7,10-tetraazacyclododecane-1,4,7-triyl}triacetate (see for example WO2022/194777, example 2).

In an embodiment of the present disclosure, the contrast agent is an agent that includes gadolinium 2,2',2"-{10-[(1R)-1-carboxy-2-{4-[2-(2-ethoxyethoxy)ethoxy]phenyl}ethyl]-1,4,7,10-tetraazacyclododecane-1,4,7-triyl}triacetate (see for example WO2022/194777, example 4).

In an embodiment of the present disclosure, the contrast agent is an agent that includes gadolinium (2S,2'S,2"S)-2,2',2"-{10-[(1S)-1-carboxy-4-{4-[2-(2-ethoxyethoxy)ethoxy]phenyl}butyl]-1,4,7,10-tetraazacyclododecane-1,4,7-triyl}tris(3-hydroxypropanoate (see for example WO2022/194777, example 15).

In an embodiment of the present disclosure, the contrast agent is an agent that includes gadolinium 2,2',2"-{10-[(1S)-4-(4-butoxyphenyl)-1-carboxybutyl]-1,4,7,10-tetraazacyclododecane-1,4,7-triyl}triacetate (see for example WO2022/194777, example 31).

In an embodiment of the present disclosure, the contrast agent is an agent that includes gadolinium - 2,2',2"-{(2S)-10-(carboxymethyl)-2-[4-(2-ethoxyethoxy)benzyl]-1,4,7,10-tetraazacyclododecane-1,4,7-triyl}triacetate.

In an embodiment of the present disclosure, the contrast agent is an agent that includes gadolinium 2,2',2"-[10-(carboxymethyl)-2-(4-ethoxybenzyl)-1,4,7,10-tetraazacyclododecane-1,4,7-triyl]triacetate.

In an embodiment of the present disclosure, the contrast agent is an agent that includes gadolinium(III) 5,8-bis(carboxylatomethyl)-2-[2-(methylamino)-2-oxoethyl]-10-oxo-2,5,8,11-tetraazadodecane-1-carboxylate hydrate (also referred to as gadodiamide).

In an embodiment of the present disclosure, the contrast agent is an agent that includes gadolinium(III) 2-[4-(2-hydroxypropyl)-7,10-bis(2-oxido-2-oxoethyl)-1,4,7,10-tetrazacyclododec-1-yl]acetate (also referred to as gadoteridol).

In an embodiment of the present disclosure, the contrast agent is an agent that includes gadolinium(III) 2,2',2"-(10-((2R,3S)-1,3,4-trihydroxybutan-2-yl)-1,4,7,10-tetraazacyclododecane-1,4,7-triyl)triacetate (also referred to as gadobutrol or Gd-DO3A-butrol).

The synthetic representation represents the examination area of the examination object after administration of a second amount of the contrast agent. The second amount is higher than the first amount.

In an embodiment of the present disclosure, the first amount is equal to or less than the standard amount. The standard amount is the amount recommended by the manufacturer and/or distributor of the contrast agent and/or the amount approved by a regulatory authority and/or the amount listed in a package insert for the contrast agent.

For example, the standard amount of Primovist^{®} is 0.025 mmol Gd-EOB-DTPA disodium/kg body weight.

In an embodiment of the present disclosure, the second amount is equal to or greater than the standard amount.

In an embodiment of the present disclosure, the first amount is less than the standard amount, and the second amount is equal to the standard amount.

In an embodiment of the present disclosure, the first amount is equal to or less than the standard amount, and the second amount is greater than the standard amount.

In an embodiment of the present disclosure, the first amount is equal to the standard amount, and the second amount is greater than the standard amount.

In an embodiment of the present disclosure, the first amount is less than the standard amount, and the second amount is greater than the standard amount.

Each representation (the synthetic representation, the native representation, and the contrast-enhanced representation) may be an image, i.e., a representation in real space; however, it may also be a representation in another space, such as a representation in frequency space or a representation in projection space.

In a representation in real space, also referred to in this disclosure as real-space representation, the examination area is normally represented by a number of image elements (for example pixels or voxels or doxels), which may for example be in a raster arrangement in which each image element represents a part of the examination area, wherein each image element may be assigned a colour value or grey value. The colour value or grey value represents a signal intensity, for example the attenuation of X-rays in case of X-ray images. A format widely used in radiology for storing and processing representations in real space is the DICOM format. DICOM (Digital Imaging and Communications in Medicine) is an open standard for storing and exchanging information in medical image data management.

In a representation in frequency space, also referred to in this description as frequency-space representation, the examination area is represented by a superposition of fundamental vibrations. For example, the examination area may be represented by a sum of sine and cosine functions having different amplitudes, frequencies, and phases. The amplitudes and phases may be plotted as a function of the frequencies, for example, in a two- or three-dimensional plot. Normally, the lowest frequency (origin) is placed in the centre. The further away from this centre, the higher the frequencies. Each frequency can be assigned an amplitude representing the frequency in the frequency-space representation and a phase indicating the extent to which the respective vibration is shifted towards a sine or cosine vibration. The k-space data produced by magnetic resonance imaging (MRI) is an example of a representation in frequency space.

A representation in real space can for example be converted (transformed) by a Fourier transform into a representation in frequency space. Conversely, a representation in frequency space can for example be converted (transformed) by an inverse Fourier transform into a representation in real space.

Details about real-space depictions and frequency-space depictions and their respective interconversion are described in numerous publications, see for example https://see.stanford.edu/materials/lsoftaee261/book-fall-07.pdf.

The representation of an examination area in the projection space can, for example, be the result of a computer tomographic examination prior to image reconstruction. In other words: the raw data obtained in the computed tomography examination can be understood as a projection-space representation. In computed tomography, the intensity or attenuation of X-ray radiation as it passes through the examination object is measured. From this, projection values can be calculated. In a second step, the object information encoded by the projection is transformed into an image (real-space depiction) through a computer-aided reconstruction. The reconstruction can be effected with the Radon transform. The Radon transform describes the link between the unknown examination object and its associated projections.

Details about the transformation of projection data into a real-space representation are described in numerous publications, see for example K. Catch: The Radon Transformation and Its Application in Tomography, Journal of Physics Conference Series 1903(1):012066.

For the sake of simplicity, the aspects of the present disclosure are described in some parts of this disclosure using the example of images as representations; however, this should not be construed as limiting the disclosure. A person skilled in the art of image processing will know how the teachings of the present disclosure can be applied to representations other than images.

The term "image" as used herein means a data structure that represents a spatial distribution of a physical signal. The spatial distribution may be of any dimension, for example 2D, 3D, 4D or any higher dimension. The spatial distribution may be of any shape, for example forming a grid and thereby defining pixels or voxels, the grid being possibly irregular or regular. The physical signal may be any signal, for example proton density, tissue echogenicity, tissue radiolucency, measurements related to the blood flow, information of rotating hydrogen nuclei in a magnetic field, color, level of gray, depth, surface or volume occupancy, such that the image may be a 2D or 3D RGB/grayscale/depth image, or a 3D surface/volume occupancy model. An image is usually composed of discrete image elements (e.g., pixels for 2D images, voxels for 3D images, doxels for 4D images).

Each representation (the synthetic representation, the native representation, and the contrast-enhanced representation) is a radiological representation. "Radiology" is the branch of medicine concerned with the application of electromagnetic radiation and mechanical waves (including, for example, ultrasound diagnostics) for diagnostic, therapeutic and/or scientific purposes. In addition to X-rays, other ionizing radiation such as gamma rays or electrons are also used. Since a primary purpose is imaging, other imaging procedures such as sonography and magnetic resonance imaging (MRI) are also included in radiology, although no ionizing radiation is used in these procedures. Thus, the term "radiology" as used in the present disclosure includes, in particular, the following examination procedures: computed tomography, magnetic resonance imaging, sonography, positron emission tomography (PET).

Each radiological representation can be, e.g., a 2D or 3D CT scan or MRI scan.

The synthetic representation is generated using a trained generative machine learning model.

Such a "machine learning model", as used herein, may be understood as a computer implemented data processing architecture. The machine learning model can receive input data and provide output data based on that input data and on parameters of the machine learning model (model parameters). The machine learning model can learn a relation between input data and output data through training. In training, parameters of the machine learning model may be adjusted in order to provide a desired output for a given input.

The process of training a machine learning model involves providing a machine learning algorithm (that is the learning algorithm) with training data to learn from. The term "trained machine learning model" refers to the model artifact that is created by the training process. The training data usually contains the correct answer, which is referred to as the target. The learning algorithm finds patterns in the training data that map input data to the target, and it outputs a trained machine learning model that captures these patterns.

In the training process, input data are inputted into the machine learning model and the machine learning model generates an output. The output is compared with the (known) target. Parameters of the machine learning model are modified in order to reduce the deviations between the output and the (known) target to a (defined) minimum.

In general, a loss function can be used for training, where the loss function can quantify the deviations between the output and the target.

The aim of the training process can be to modify (adjust) parameters of the machine learning model in order to reduce the loss to a (defined) minimum.

The deviations between output and target can be reduced in an optimization procedure, e.g. a gradient descent procedure.

A "generative machine learning model" is a type of machine learning model that is designed to learn and generate new data that resembles the training data it was trained on.

The generative machine learning model can be or comprise an artificial neural network, for example.

An "artificial neural network" (ANN) is a computational model inspired by the structure and functional aspects of biological neural networks. It consists of interconnected processing elements, known as neurons, which usually work in unison to solve specific problems.

An ANN comprises at least three layers of processing elements: a first layer having input neurons, an N-th layer having output neurons and N - 2 inner layers, where N is a natural number and greater than 2.

The input neurons serve to receive the input data (e.g., a first and/or second (reference) representation). There may for example be one input neuron for each image element of a (reference) representation when the representation is a real-space depiction in the form of a raster graphic, or one input neuron for each frequency present in the (reference) representation when the representation is a frequency-space depiction. There may be additional input neurons for additional input data (for example information about the examination area, about the examination object/reference object, about the conditions prevailing during the generation of the input representation(s), information about the state that the (reference) representation represents, information about the time or time interval at/during which the (reference) representation had been generated and/or information about the contrast agent (e.g. type and/or amount of contrast agent)).

The output neurons serve to output the (first) synthetic representation.

The processing elements of the layers between the input neurons and the output neurons are connected to one another in a predetermined pattern with predetermined connection weights.

The artificial neural network may be a convolutional neural network (CNN for short) or include such a network.

A convolutional neural network is capable of processing input data in the form of a matrix. This makes it possible to use as input data digital radiological images depicted in the form of a matrix (e.g. width x height x colour channels). A normal neural network, for example in the form of a multilayer perceptron (MLP), requires on the other hand a vector as input, i.e. in order to use a radiological image as input, the pixels or voxels of the radiological image would have to be rolled out in a long chain one after the other. This means that normal neural networks are for example not able to recognize objects in a radiological image independently of the position of the object in the image. The same object at a different position in the image would have a completely different input vector.

A CNN normally consists essentially of an alternately repeating array of filters (convolutional layer) and aggregation layers (pooling layer) terminating in one or more layers of fully connected neurons (dense/fully connected layer).

The generative machine learning model may be or comprise a generative adversarial network (GAN). GANs are class of machine learning models where two neural networks, the generator and the discriminator, are trained simultaneously through adversarial processes. The generator learns to produce content (in this case, synthetic representations) that is indistinguishable from real data, while the discriminator learns to distinguish genuine data from the content created by the generator.

The generative machine learning model may be or comprise a variational autoencoder (VAE).

The generative machine learning model may be or comprise a transformer. Originally designed for natural language processing tasks, transformers have been adapted for image generation. These models utilize attention mechanisms to generate images by understanding the context and relationships between different parts of an image.

The generative machine learning model may be or comprise a diffusion model.

Diffusion models focus on modeling the step-by-step evolution of a data distribution from a "simple" starting point to a "more complex" distribution. The underlying concept of diffusion models is to transform a simple and easily sampleable distribution, typically a Gaussian distribution, into a more complex data distribution of interest. This transformation is achieved through a series of invertible operations. Once the model learns the transformation process, it can generate new samples by starting from a point in the simple distribution and gradually "diffusing" it to the desired complex data distribution.

The generative machine learning model may have been pre-trained. The term "pre-trained" refers to a model that has been trained on a training dataset. Pre-training often involves training a model on a task or dataset that is different from the specific task for which the model will be used later. The pre-training process involves exposing the model to a vast amount of data and allowing it to learn general patterns and representations from that data. This enables the model to capture common features and structures that are useful across various related tasks. The model is typically trained using unsupervised or self-supervised learning methods, where the target data are generated automatically or do not require human intervention.

The generative machine learning model of the present disclosure is configured and trained to generate a synthetic representation of an examination area based on one or more representations of the examination area.

The generative machine learning model is trained on training data. The training data comprises, for each reference object of a plurality of reference objects, a first reference representation of the examination area of the reference object, and a second reference representation of the examination area of reference object.

The term "plurality" means more than ten, e.g., more than a hundred or even more than a thousand.

The term "reference" is used in this description to distinguish the phase of training the generative machine learning model from the phase of using the trained model for generating a synthetic representation. The term "reference" otherwise has no limitation on meaning. A "reference object" is an object from which data was used to train the generative machine learning model. A "reference representation" is a representation of the examination area of a reference object which is used to train the generative machine learning model. An "examination object" is an object from which data is used to generate a synthetic representation of the examination area of the examination object using the trained generative machine learning model. Everything described in this disclosure for an examination object also applies analogously to any reference object and *vice versa.* Each reference object is therefore a living being, e.g. a mammal, e.g. a human being. The examination area is part of the reference object, e.g., a liver, kidney, heart, lung, brain, stomach, bladder, prostate, intestine, breast, thyroid, pancreas, uterus or a part of said parts or another part of the examination object.

The examination area is usually the same for all reference objects as well as for the examination object.

Each reference representation can be a representation in real space (image space, spatial domain), a representation in frequency space (frequency domain), a representation in the projection space or a representation in another space, as described above.

The first reference representation is a native representation of the examination area of the reference object. The first reference representation represents the examination area of the reference object without contrast agent. The second reference representation of the examination area of reference object is a contrast-enhanced representation. The second reference representation represents the examination area of reference object after application of the first amount of the contrast agent.

During training, the generative machine learning model is caused to generate a synthetic representation for each reference object. The generative machine learning model is trained to generate a synthetic representation of the examination area of the reference object that represents the examination area of the reference object after application of the second amount of the contrast agent.

The synthetic representation can be generated based on the first representation and/or the second representation. The synthetic representation can be generated based on further data.

The generative machine learning model is caused to generate a first synthetic representation of the examination area of the reference object based on the first reference representation and/or the second reference representation.

The first synthetic representation may be received via an output of the generative machine learning model.

Based on the first synthetic representation, a second synthetic representation of the examination area of the reference object is generated.

Generating the second representation comprises: subtracting the first reference representation from the first synthetic representation.

Subtraction can be carried out in real space, for example. When subtracting in real space, the grey values or color values of corresponding image elements are subtracted from each other. Corresponding image elements are those that represent the same sub-area of the examination area. However, subtraction can also take place in a different space, e.g. in frequency space.

The result of this subtraction is a representation of the examination area of the reference object in which the contrast is at least mainly caused by the distribution of the contrast agent in the examination area.

The second synthetic representation can be the direct result of the subtraction. It is possible to set negative values that may occur during subtraction to zero or another value. Further/other steps are possible.

Based on the second synthetic representation, a third synthetic representation of the examination area of the reference object is generated.

Generating the third synthetic representation comprises: reducing the contrast of the second synthetic representation. The contrast reduction can be linear or non-linear.

The contrast can be reduced in real space, for example, by multiplying the grey values or color values by an attenuation factor, whereby the attenuation factor is greater than zero and less than one, for example.

Such a contrast reduction can be a linear contrast reduction: all gray values or color values of the second synthetic representation are multiplied by the same attenuation factor. However, the contrast reduction can also be non-linear; it is possible, for example, that the attenuation factor depends on the size of the grey value/color value and/or on the coordinates of the image element and/or on grey values/color values of neighboring image elements.

The attenuation factor can be used to control the ratio between the first amount and the second amount of the contrast agent. The attenuation factor may correspond to the ratio of the first amount to the second amount. This means that with an attenuation factor of 1/2, the second amount is about twice as high as the first amount. With an attenuation factor of 1/3, the second amount is about three times as large as the first amount.

The attenuation factor may be in the range of 0.1 to 0.9, for example. **In** an embodiment of the present disclosure, the attenuation factor is in the range of 0.25 to 0.85. **In** an embodiment of the present disclosure, the attenuation factor is in the range of 0.4 to 0.84.

It is possible that the third synthetic representation is the result of multiplying the grey values/color values by one or more attenuation factors. It is possible that the result of the multiplication of the grey values/color values with one or more attenuation factors is subjected to filtering. Such filtering can, for example, be a frequency-dependent weighting in frequency space. **In** such a case, a frequency space representation can be multiplied by a frequency-dependent weighting function. For example, low frequencies can be weighted higher than high frequencies or high frequencies lower than low frequencies. Contrast information is represented in a frequency-space representation by low frequencies, while the higher frequencies represent information about fine structures. Preferred weight functions are Hann function (also referred to as the Hann window) and Poisson function (Poisson window). Examples of other weight functions can be found for example at https://de.wikipedia.org/wiki/Fensterfunktion#Beispiele_von_Fensterfunktionen; F.J. Harris et al.: On the Use of Windows for Harmonic Analysis with the Discrete Fourier Transform, Proceedings of the IEEE, vol. 66, No. 1, 1978; https://docs.scipy.org/doc/scipy/reference/signal.windows.html; K. M. M Prabhu: Window Functions and Their Applications in Signal Processing, CRC Press, 2014, 978-1-4665-1583-3), WO2024/052156A1.

Based on the third synthetic representation, a fourth synthetic representation of the examination area of the reference object is generated. Generating the fourth synthetic representation comprises: subtracting the third synthetic representation from the first synthetic representation or adding the third synthetic representation to the first reference representation. The addition can be carried out in real space or frequency space, for example. If it is carried out in real space, the grey values/color values of corresponding image elements are added together. The result of the addition can be subjected to filtering, e.g. filtering as described above.

The fourth synthetic representation is a representation in which the contrast enhancement is reduced compared to the first synthetic representation. The fourth synthetic representation is a representation in which the contrast enhancement by contrast agent in the examination area is lower than in the first synthetic representation. The fourth synthetic representation is intended to represent the examination area of the examination object in the same way as the second reference representation. The transformations performed from the first synthetic representation to the fourth synthetic representation are intended to restore what the generative machine learning model has produced.

The fourth synthetic representation is compared with the second reference representation. The second reference representation therefore serves as the target. Deviations between the fourth synthetic representation and the second reference representation are quantified. The deviations can be quantified using a loss function. The deviations can be reduced by modifying parameters of the generative machine learning model. This can be done in an optimization process, e.g. in a gradient descent procedure.

The training of the generative machine leaning model can be ended when a stop criterion is met. Such a stop criterion can be for example: a predefined maximum number of training steps/cycles/epochs has been performed, deviations between output data and target data can no longer be reduced by modifying model parameters, a predefined minimum of the loss function is reached, and/or an extreme value (e.g., maximum or minimum) of another performance value is reached.

The trained generative machine learning model can be output, stored in a data memory and/or transmitted to another computer system.

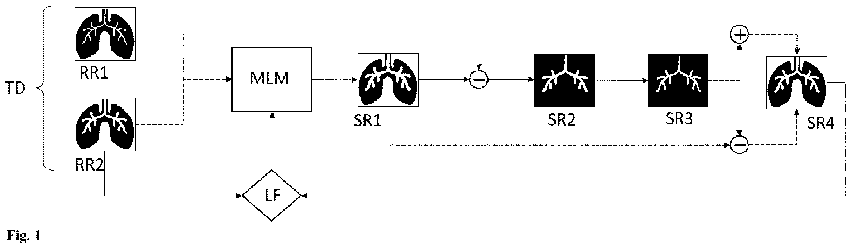

Fig. 1 shows schematically an example of training a generative machine learning model.

The training takes place based on training data TD. The training data TD comprise for each reference object of a multiplicity of reference objects: (i) a first reference representation RR1 of an examination area of the reference object and a second reference representation RR2 of the examination area of the reference object. Fig. 1 shows only a single data set for a single reference object. The reference object is a human, and the examination area includes the lung of the human.

The first reference representation RR1 represents the examination area of the reference object without contrast agent. The second reference representation RR2 represents the examination area of the reference object after application of a first amount of a contrast agent.

The first reference representation RR1 and/or the second reference representation RR2 are fed into the generative machine learning model MLM. It is possible for further data to be fed into the generative machine learning model MLM. It is possible that instead of or in addition to the first reference representation RR1 and/or the second reference representation RR2 the difference between the first reference representation RR1 and the second reference representation RR2 is fed to the MLM machine learning model. It is possible that the machine learning model is configured and trained to increase the contrast enhancement based on difference representations.

The generative machine learning model MLM is configured to generate a first synthetic representation SR1 of the examination area of the reference object based on the first reference representation RR1 and/or the second reference representation RR2 and optionally further data.

Based on the first synthetic representation SR1, a second synthetic representation SR2 is generated. Generation of the second synthetic representation SR2 comprises: subtracting the first reference representation RR1 from the first synthetic representation SR1 (SR1-RR1).

Based on the second synthetic representation SR2, a third synthetic representation SR3 is generated. Generation of the third synthetic representation SR3 comprises: reducing the contrast of the second synthetic representation SR2. For example, the grey values/color values of the image elements of the second synthetic representation SR2 can be multiplied by an attenuation factor, whereby the attenuation factor can be greater than zero and less than one. Other methods for reducing the contrast are conceivable.

Based on the third synthetic representation SR3, a fourth synthetic representation SR4 is generated. Generation of the fourth synthetic representation SR4 comprises: subtracting the third synthetic representation SR3 from the first synthetic representation SR1 (SR1-SR3) or adding the third synthetic representation SR3 to the first reference representation RR1 (RR1+SR3).

Deviations between the synthetic fourth representation SR4 and the second reference representation RR2 are quantified using a loss function LF.

The deviations can be reduced by modifying parameters of the generative machine learning model MLM, e.g., in an optimization process.

In the example shown in Fig. 1, all steps are carried out in real space. It should be noted that at least some of the steps can also be carried out in another space, e.g. in frequency space.

The trained generative machine learning model can be used to generate a synthetic representation of the examination area of an examination object.

The generation of the synthetic representation is based on a first representation of the examination area of the examination object and/or on a second representation of the examination area of the examination object. The first representation represents the examination area of the examination object without contrast agent; the second representation represents the examination area of the examination object after application of the first amount of contrast agent.

The generation of the synthetic representation can be based on a difference between the first representation and the second representation. The generation of the synthetic representation can be based on further data.

The synthetic representation represents the examination area after application of the second amount of the contrast agent. The second amount is larger than the first amount.

The synthetic representation of the examination area of the examination object can be output, e.g., displayed on a monitor and/or printed using a printer. The synthetic representation of the examination area of the examination object can be stored in a data storage. The synthetic representation of the examination area of the examination object can be transmitted to a separate computer system.

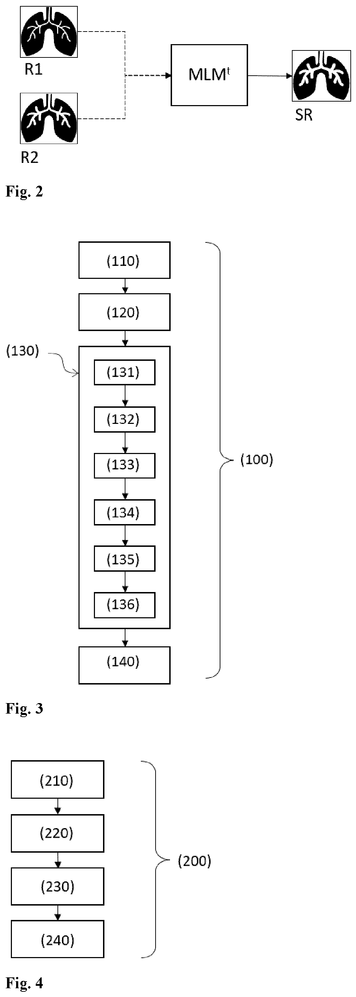

Fig. 2 exemplarily and schematically illustrates the generation of a synthetic representation of the examination area of an examination object.

The synthetic representation SR is generated using a trained generative machine learning model MLM^{t}. The trained generative machine learning model MLM^{t} may have been trained as described in relation to Fig. 1.

The trained generative machine learning model MLM^{t} is fed a first representation R1 and/or a second representation R2 and/or data derived from the first representation R1 and/or a second representation R2 and optionally further data (depending on how the model has been trained).

The first representation R1 represents the examination area of the examination object without contrast agent; the second representation R2 represents the examination area of the examination object after application of the first amount of contrast agent. The examination area comprises the lungs of a human.

The trained generative machine learning model MLM^{t} outputs the synthetic representation SR of the examination area of the examination object. The synthetic representation SR represents the examination area after application of the second amount of the contrast agent. The second amount is larger than the first amount.

Fig. 3 shows an embodiment of the method of training the machine learning model in the form of a flow chart.

The training method (100) comprises the steps:
- (110): providing a generative machine learning model,
- (120): providing training data, wherein the training data comprises, for each reference object of a plurality of reference objects, a first reference representation representing an examination area of the reference object without contrast agent, and a second reference representation representing the examination area of the reference object after application of an amount of a contrast agent,
- (130): training the generative machine learning model, wherein training the generative machine learning model comprises, for each reference object of the plurality of reference objects:
(131) causing the generative machine learning model to generate a first synthetic representation of the examination area of the reference object based on the first reference representation and/or the second reference representation,
(132) generating a second synthetic representation of the examination area of the reference object, wherein generating the second synthetic representation comprises: subtracting the first reference representation from the first synthetic representation,
(133) generating a third synthetic representation of the examination area of the reference object, wherein generating the third synthetic representation comprises: reducing the contrast of the second synthetic representation,
(134) generating a fourth synthetic representation of the examination area of the reference object, wherein generating the fourth synthetic representation comprises: subtracting the third synthetic representation from the first synthetic representation or adding the third synthetic representation to the first reference representation,
(135) determining deviations between the fourth synthetic representation and the second reference representation,
(136) reducing the deviations by modifying parameters of the generative machine learning model,
- (140): storing the trained generative machine learning model and/or transmitting the trained generative machine learning model to another computer system and/or using the trained generative machine learning model to generate a synthetic representation of the examination area of an examination object.

Fig. 4 shows an embodiment of the method of generating a synthetic representation in the form of a flow chart.

The method (200) comprises the steps:
- (210): providing a trained generative machine learning model,
- wherein the trained generative machine learning model was trained on training data,
- wherein the training data comprised, for each reference object of a plurality of reference objects, a first reference representation representing an examination area of the reference object without contrast agent, and a second reference representation representing the examination area of the reference object after application of an amount of a contrast agent,
- wherein training the generative machine learning model comprised, for each reference object of the plurality of reference objects:
   ∘ causing the generative machine learning model to generate a first synthetic representation of the examination area of the reference object based on the first reference representation and/or the second reference representation,
   ∘ generating a second synthetic representation of the examination area of the reference object, wherein generating the second synthetic representation comprises: subtracting the first reference representation from the first synthetic representation,
   ∘ generating a third synthetic representation of the examination area of the reference object, wherein generating the third synthetic representation comprises: reducing the contrast of the second synthetic representation,
   ∘ generating a fourth synthetic representation of the examination area of the reference object, wherein generating the fourth synthetic representation comprises: subtracting the third synthetic representation from the first synthetic representation or adding the third synthetic representation to the first reference representation,
   ∘ determining deviations between the fourth synthetic representation and the second reference representation,
   ∘ reducing the deviations by modifying parameters of the generative machine learning model,
- (220): providing a first representation and/or a second representation, wherein the first representation represents the examination area of an examination object without contrast agent, and the second representation represents the examination area of the examination object after application of the amount of the contrast agent,
- (230): causing the trained generative machine learning model to generate a synthetic representation of the examination area of the examination object based on the first representation and/or the second representation,
- (240): outputting the synthetic representation of the examination area of the examination object and/or storing the synthetic representation of the examination area of the examination object in a data storage and/or transmitting the synthetic representation of the examination area of the examination object to a separate computer system.

The operations in accordance with the teachings herein may be performed by at least one computer specially constructed for the desired purposes or general-purpose computer specially configured for the desired purpose by at least one computer program stored in a typically non-transitory computer readable storage medium.

The term "non-transitory" is used herein to exclude transitory, propagating signals or waves, but to otherwise include any volatile or non-volatile computer memory technology suitable to the application.

The term "computer" should be broadly construed to cover any kind of electronic device with data processing capabilities, including, by way of non-limiting example, personal computers, servers, embedded cores, computing system, communication devices, processors (e.g., digital signal processor (DSP)), microcontrollers, field programmable gate array (FPGA), application specific integrated circuit (ASIC), etc.) and other electronic computing devices.

The term "process" as used above is intended to include any type of computation or manipulation or transformation of data represented as physical, e.g., electronic, phenomena which may occur or reside e.g., within registers and/or memories of at least one computer or processor. The term processor includes a single processing unit or a plurality of distributed or remote such units.

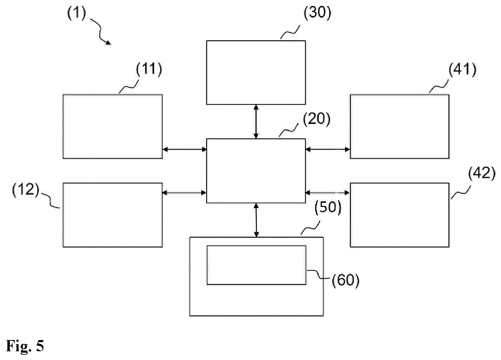

Fig. 5 illustrates a computer system (1) according to some example implementations of the present disclosure in more detail. The computer may include one or more of each of a number of components such as, for example, processing unit (20) connected to a memory (50) (e.g., storage device).

The processing unit (20) may be composed of one or more processors alone or in combination with one or more memories. The processing unit is generally any piece of computer hardware that is capable of processing information such as, for example, data, computer programs and/or other suitable electronic information. The processing unit is composed of a collection of electronic circuits some of which may be packaged as an integrated circuit or multiple interconnected integrated circuits (an integrated circuit at times more commonly referred to as a "chip"). The processing unit may be configured to execute computer programs, which may be stored onboard the processing unit or otherwise stored in the memory (50) of the same or another computer.

The processing unit (20) may be a number of processors, a multi-core processor or some other type of processor, depending on the particular implementation. Further, the processing unit may be implemented using a number of heterogeneous processor systems in which a main processor is present with one or more secondary processors on a single chip. As another illustrative example, the processing unit may be a symmetric multi-processor system containing multiple processors of the same type. In yet another example, the processing unit may be embodied as or otherwise include one or more ASICs, FPGAs or the like. Thus, although the processing unit may be capable of executing a computer program to perform one or more functions, the processing unit of various examples may be capable of performing one or more functions without the aid of a computer program. In either instance, the processing unit may be appropriately programmed to perform functions or operations according to example implementations of the present disclosure.

The memory (50) is generally any piece of computer hardware that is capable of storing information such as, for example, data, computer programs (e.g., computer-readable program code (60)) and/or other suitable information either on a temporary basis and/or a permanent basis. The memory may include volatile and/or non-volatile memory, and may be fixed or removable. Examples of suitable memory include random access memory (RAM), read-only memory (ROM), a hard drive, a flash memory, a thumb drive, a removable computer diskette, an optical disk, a magnetic tape or some combination of the above. Optical disks may include compact disk - read only memory (CD-ROM), compact disk - read/write (CD-R/W), DVD, Blu-ray disk or the like. In various instances, the memory may be referred to as a computer-readable storage medium. The computer-readable storage medium is a non-transitory device capable of storing information, and is distinguishable from computer-readable transmission media such as electronic transitory signals capable of carrying information from one location to another. Computer-readable medium as described herein may generally refer to a computer-readable storage medium or computer-readable transmission medium.

In addition to the memory (50), the processing unit (20) may also be connected to one or more interfaces for displaying, transmitting and/or receiving information. The interfaces may include one or more communications interfaces and/or one or more user interfaces. The communications interface(s) may be configured to transmit and/or receive information, such as to and/or from other computer(s), network(s), database(s) or the like. The communications interface may be configured to transmit and/or receive information by physical (wired) and/or wireless communications links. The communications interface(s) may include interface(s) (41) to connect to a network, such as using technologies such as cellular telephone, Wi-Fi, satellite, cable, digital subscriber line (DSL), fiber optics and the like. In some examples, the communications interface(s) may include one or more short-range communications interfaces (42) configured to connect devices using short-range communications technologies such as NFC, RFID, Bluetooth, Bluetooth LE, ZigBee, infrared (e.g., IrDA) or the like.

The user interfaces may include a display (30). The display may be configured to present or otherwise display information to a user, suitable examples of which include a liquid crystal display (LCD), light-emitting diode display (LED), plasma display panel (PDP) or the like. The user input interface(s) (11) may be wired or wireless, and may be configured to receive information from a user into the computer system (1), such as for processing, storage and/or display. Suitable examples of user input interfaces include a microphone, image or video capture device, keyboard or keypad, joystick, touch-sensitive surface (separate from or integrated into a touchscreen) or the like. In some examples, the user interfaces may include automatic identification and data capture (AIDC) technology (12) for machine-readable information. This may include barcode, radio frequency identification (RFID), magnetic stripes, optical character recognition (OCR), integrated circuit card (ICC), and the like. The user interfaces may further include one or more interfaces for communicating with peripherals such as printers and the like.

As indicated above, program code instructions may be stored in memory, and executed by processing unit that is thereby programmed, to implement functions of the systems, subsystems, tools and their respective elements described herein. As will be appreciated, any suitable program code instructions may be loaded onto a computer or other programmable apparatus from a computer-readable storage medium to produce a particular machine, such that the particular machine becomes a means for implementing the functions specified herein. These program code instructions may also be stored in a computer-readable storage medium that can direct a computer, processing unit or other programmable apparatus to function in a particular manner to thereby generate a particular machine or particular article of manufacture. The instructions stored in the computer-readable storage medium may produce an article of manufacture, where the article of manufacture becomes a means for implementing functions described herein. The program code instructions may be retrieved from a computer-readable storage medium and loaded into a computer, processing unit or other programmable apparatus to configure the computer, processing unit or other programmable apparatus to execute operations to be performed on or by the computer, processing unit or other programmable apparatus.

Retrieval, loading and execution of the program code instructions may be performed sequentially such that one instruction is retrieved, loaded and executed at a time. In some example implementations, retrieval, loading and/or execution may be performed in parallel such that multiple instructions are retrieved, loaded, and/or executed together. Execution of the program code instructions may produce a computer-implemented process such that the instructions executed by the computer, processing circuitry or other programmable apparatus provide operations for implementing functions described herein.

Execution of instructions by processing unit, or storage of instructions in a computer-readable storage medium, supports combinations of operations for performing the specified functions. In this manner, a computer system (1) may include processing unit (20) and a computer-readable storage medium or memory (50) coupled to the processing circuitry, where the processing circuitry is configured to execute computer-readable program code (60) stored in the memory. It will also be understood that one or more functions, and combinations of functions, may be implemented by special purpose hardware-based computer systems and/or processing circuitry which perform the specified functions, or combinations of special purpose hardware and program code instructions.

The computer-readable program code of the present disclosure may also be marketed in combination with a contrast agent. Such a combination is also referred to as a kit. Such a kit includes the contrast agent and the computer-readable program code. It is also possible that such a kit includes the contrast agent and means for allowing a purchaser to obtain the computer-readable program code, e.g., download it from an Internet site. These means may include a link, i.e., an address of the Internet site from which the computer-readable program code may be obtained, e.g., from which the computer-readable program code may be downloaded to a computer system connected to the Internet. Such means may include a code (e.g., an alphanumeric string or a QR code, or a DataMatrix code or a barcode or other optically and/or electronically readable code) by which the purchaser can access the computer-readable program code. Such a link and/or code may, for example, be printed on a package of the contrast agent and/or printed on a package insert for the contrast agent. A kit is thus a combination product comprising a contrast agent and a computer-readable program code (e.g., in the form of access to the computer-readable program code or in the form of executable program code on a data carrier) that is offered for sale together.

The aspects of the present disclosure can be used for various purposes. Some examples of use are described below, without the disclosure being limited to these examples of use.

A first example of use concerns magnetic resonance imaging examinations for differentiating intraaxial tumours such as intracerebral metastases and malignant gliomas. The infiltrative growth of these tumours makes it difficult to differentiate exactly between tumour and healthy tissue. Determining the extent of a tumour is however crucial for surgical removal. Distinguishing between tumours and healthy tissue is facilitated by administration of an extracellular MRI contrast agent; after intravenous administration of a standard dose of 0.1 mmol/kg body weight of the extracellular MRI contrast agent gadobutrol, intraaxial tumours can be differentiated much more readily. At higher doses, the contrast between lesion and healthy brain tissue is increased further; the detection rate of brain metastases increases linearly with the dose of the contrast agent (see for example M. Hartmann et al.: Does the administration of a high dose of a paramagnetic contrast medium (Gadovist) improve the diagnostic value of magnetic resonance tomography in glioblastomas? doi: 10.1055/s-2007-1015623).

A single triple dose or a second subsequent dose may be administered here up to a total dose of 0.3 mmol/kg body weight. This exposes the patient and the environment to additional gadolinium and in the case of a second scan, incurs additional costs.

The aspects of the present disclosure can be used to avoid the dose of contrast agent exceeding the standard amount. A first MRI image can be generated without contrast agent or with less than the standard amount and a second MRI image generated with the standard amount. On the basis of these generated MRI images it is possible, as described in this disclosure, to generate a synthetic MRI image in which the contrast between lesions and healthy tissue can be varied within wide limits. This makes it possible to achieve contrasts that are otherwise achievable only by administering an amount of contrast agent larger than the standard amount.

Another example of use concerns the reduction of the amount of MRI contrast agent in a magnetic resonance imaging examination. Gadolinium-containing contrast agents such as gadobutrol are used for a diversity of examinations. They are used for contrast enhancement in examinations of the cranium, spine, chest or other examinations. In the central nervous system, gadobutrol highlights regions where the blood-brain barrier is impaired and/or abnormal vessels. In breast tissue, gadobutrol makes it possible to visualize the presence and extent of malignant breast disease. Gadobutrol is also used in contrast-enhanced magnetic resonance angiography for diagnosing stroke, for detecting tumour blood perfusion and for detecting focal cerebral ischaemia.

Increasing environmental pollution, the cost burden on the health system and the fear of acute side effects and possible long-term health risks, especially in the case of repeated and long-term exposure, have given impetus to efforts to reduce the dose of gadolinium-containing contrast agents. This can be achieved by the aspects of the present disclosure.

A first MRI image without contrast agent and a second MRI image with an amount of contrast agent less than the standard amount can be generated. On the basis of these generated MRI images it is possible, as described in this disclosure, to generate a synthetic MRI image in which the contrast can be varied within wide limits. This makes it possible with less than the standard amount of contrast agent to achieve the same contrast as is obtained after administration of the standard amount.

Another example of use concerns the detection, identification and/or characterization of lesions in the liver with the aid of a hepatobiliary contrast agent such as Primovist^{®}.

Primovist^{®} is administered intravenously (i.v.) at a standard dose of 0.025 mmol/kg body weight. This standard dose is lower than the standard dose of 0.1 mmol/kg body weight in the case of extracellular MRI contrast agents. Unlike in contrast-enhanced MRI with extracellular gadolinium-containing contrast agents, Primovist^{®} permits dynamic multiphase T1w imaging. However, the lower dose of Primovist^{®} and the observation of transient motion artefacts that can occur shortly after intravenous administration, means that contrast enhancement with Primovist^{®} in the arterial phase is perceived by radiologists as poorer than contrast enhancement with extracellular MRI contrast agents. The assessment of contrast enhancement in the arterial phase and of the vascularity of focal liver lesions is however of critical importance for accurate characterization of the lesion.

With the aid of the present disclosure it is possible to increase contrast, particularly in the arterial phase, without the need to administer a higher dose.

A first MRI image without contrast agent and a second MRI image during the arterial phase after administering an amount of a contrast agent that corresponds to the standard amount can be generated. On the basis of these generated MRI images it is possible, as described in this disclosure, to generate a synthetic MRI image in which the contrast in the arterial phase can be varied within wide limits. This makes it possible to achieve contrasts that are otherwise achievable only by administering an amount of contrast agent larger than the standard amount.

Another example of use concerns the use of MRI contrast agents in computed tomography examinations.

In a CT examination, MRI contrast agents usually have a lower contrast-enhancing effect than CT contrast agents. However, it can be advantageous to employ an MRI contrast agent in a CT examination. An example is a minimally invasive intervention in the liver of a patient in whom a surgeon is monitoring the procedure by means of a CT scanner. Computed tomography (CT) has the advantage over magnetic resonance imaging that more major surgical interventions are possible in the examination area while generating CT images of an examination area of an examination object. On the other hand, there are only few surgical instruments and surgical devices that are MRI-compatible. Moreover, access to the patient is restricted by the magnets used in MRI. Thus, while a surgeon is performing an operation in the examination area, he/she is able to use CT to visualize the examination area and to monitor the operation.

For example, if a surgeon wishes to perform a procedure in a patient's liver in order for example to carry out a biopsy on a liver lesion or to remove a tumour, the contrast between a liver lesion or tumour and healthy liver tissue will not be as pronounced in a CT image of the liver as it is in an MRI image after administration of a hepatobiliary contrast agent. No CT-specific hepatobiliary contrast agents are currently known and/or approved in CT. The use of an MRI contrast agent, more particularly a hepatobiliary MRI contrast agent, in computed tomography thus combines the possibility of differentiating between healthy and diseased liver tissue and the possibility of carrying out an operation with simultaneous visualization of the liver.

The comparatively low contrast enhancement achieved by the MRI contrast agent can be increased with the aid of the present disclosure without the need to administer a dose higher than the standard dose.

A first CT image without MRI contrast agent and a second CT image after administering an amount of a MRI contrast agent that corresponds to the standard amount can be generated. On the basis of these generated CT images it is possible, as described in this disclosure, to generate a synthetic CT image in which the contrast produced by the MRI contrast agent can be varied within wide limits. This makes it possible to achieve contrasts that are otherwise achievable only by administering an amount of MRI contrast agent larger than the standard amount.

## Claims

1. A computer-implemented method of training a generative machine learning model (MLM), the method comprising:
- providing the generative machine learning model (MLM),
- providing training data (TD), wherein the training data (TD) comprises, for each reference object of a plurality of reference objects, a first reference representation (RR1) representing an examination area of the reference object without contrast agent, and a second reference representation (RR2) representing the examination area of the reference object after application of a first amount of a contrast agent,
- training the generative machine learning model (MLM), wherein training the generative machine learning model (MLM) comprises, for each reference object of the plurality of reference objects:
• causing the generative machine learning model (MLM) to generate a first synthetic representation (SR1) of the examination area of the reference object based on the first reference representation (RR1) and/or the second reference representation (RR2),
• generating a second synthetic representation (SR2) of the examination area of the reference object, wherein generating the second synthetic representation (SR2) comprises: subtracting the first reference representation (RR1) from the first synthetic representation (SR1),
• generating a third synthetic representation (SR3) of the examination area of the reference object, wherein generating the third synthetic representation (SR3) comprises: reducing the contrast of the second synthetic representation (SR2),
• generating a fourth synthetic representation (SR4) of the examination area of the reference object, wherein generating the fourth synthetic representation (SR4) comprises: subtracting the third synthetic representation (SR3) from the first synthetic representation (SR1) or adding the third synthetic representation (SR3) to the first reference representation (RR1),
• determining deviations between the fourth synthetic representation (SR4) and the second reference representation (RR2),
• reducing the deviations by modifying parameters of the generative machine learning model (MLM),
- storing the trained generative machine learning model (MLM^{t}) and/or transmitting the trained generative machine learning model (MLM^{t}) to another computer system and/or using the trained generative machine learning model (MLM^{t}) to generate a synthetic representation (SR) of the examination area of an examination object.

2. A computer-implemented method of generating a synthetic representation (SR) using a trained generative machine learning model (MLM^{t}), the method comprising:
- providing the trained generative machine learning model (MLM^{t}),
• wherein the trained generative machine learning model (MLM^{t}) was trained on training data (TD),
• wherein the training data (TD) comprised, for each reference object of a plurality of reference objects, a first reference representation (RR1) representing an examination area of the reference object without contrast agent, and a second reference representation (RR2) representing the examination area of the reference object after application of a first amount of a contrast agent,
• wherein training the generative machine learning model (MLM) comprised, for each reference object of the plurality of reference objects:
∘ causing the generative machine learning model (MLM) to generate a first synthetic representation (SR1) of the examination area of the reference object based on the first reference representation (RR1) and/or the second reference representation (RR2),
∘ generating a second synthetic representation (SR2) of the examination area of the reference object, wherein generating the second synthetic representation (SR2) comprises: subtracting the first reference representation (RR1) from the first synthetic representation (SR1),
∘ generating a third synthetic representation (SR3) of the examination area of the reference object, wherein generating the third synthetic representation (SR3) comprises: reducing the contrast of the second synthetic representation (SR2),
∘ generating a fourth synthetic representation (SR4) of the examination area of the reference object, wherein generating the fourth synthetic representation (SR4) comprises: subtracting the third synthetic representation (SR3) from the first synthetic representation (SR1) or adding the third synthetic representation (SR3) to the first reference representation (RR1),
∘ determining deviations between the fourth synthetic representation (SR4) and the second reference representation (RR2),
∘ reducing the deviations by modifying parameters of the generative machine learning model (MLM),
- providing a first representation (R1) and/or a second representation (R2) of the examination area of an examination object, wherein the first representation (R1) represents the examination area of the examination object without contrast agent, and the second representation (R2) represents the examination area of the examination object after application of the first amount of the contrast agent,
- causing the trained generative machine learning model (MLM^{t}) to generate a synthetic representation (SR) of the examination area of the examination object based on the first representation (R1) and/or the second representation (R2),
- outputting the synthetic representation (SR) of the examination area of the examination object and/or storing the synthetic representation (SR) of the examination area of the examination object in a data storage and/or transmitting the synthetic representation (SR) of the examination area of the examination object to a separate computer system.

3. The method of claim 1 or 2, wherein each reference object is a living being, e.g. a mammal, e.g. a human, and the examination object is a living being, e.g. a mammal, e.g. a human.

4. The method of any one of claims 1 to 3, wherein the examination area is or comprises a liver, kidney, heart, lung, brain, stomach, bladder, prostate, intestine, breast, thyroid, pancreas, uterus or a part thereof of a mammal, e.g. of a human.

5. The method of any one of claims 1 to 4, wherein each representation is a radiologic representation.

6. The method of any one of claims 1 to 5, wherein the synthetic representation (SR) of the examination area of the examination object represents the examination area of the examination object after application of a second amount of the contrast agent, wherein the second amount is larger than the first amount.

7. The method of any one of claims 1 to 6, wherein reducing the contrast of the second synthetic representation (SR2) comprises: linear or non-linear attenuation of grey values or color values of image elements of the second synthetic representation (SR2)

8. The method of any one of claims 1 to 7, wherein reducing the contrast of the second synthetic representation (SR2) comprises: multiplying grey values or color values of image elements of the second synthetic representation (SR2) by an attenuation factor, wherein the attenuation factor is greater than zero and smaller than 1.

9. The method of any one of claims 1 to 8, wherein the generative machine learning model (MLM) is or comprises one or more of the following: artificial neural network, convolutional neural network, variational autoencoder, generative adversarial network, transformer, diffusion network.

10. The method of any one of claims 1 to 10, wherein the examination area is a human liver or comprises a human liver or is part of a human liver, and the contrast agent is a hepatobiliary contrast agent, e.g. a hepatobiliary MRI contrast agent.

11. The method of any one of claims 1 to 11, wherein the generation of the first synthetic representation (SR1) of the examination area of the reference object is based on the first reference representation (RR1) and the second reference representation (RR2), and the generation of the synthetic representation (SR) of the examination area of the examination object is based on the first representation (R1) and the second representation (R2).

12. A computer system (1) comprising:
a processing unit (20) or; and
a memory (50) storing a computer program (60) configured to perform, when executed by the processing unit (20) or, an operation, the operation comprising:
- providing the trained generative machine learning model (MLM^{t}),
• wherein the trained generative machine learning model (MLM^{t}) was trained on training data (TD),
• wherein the training data (TD) comprised, for each reference object of a plurality of reference objects, a first reference representation (RR1) representing an examination area of the reference object without contrast agent, and a second reference representation (RR2) representing the examination area of the reference object after application of a first amount of a contrast agent,
• wherein training the generative machine learning model (MLM) comprised, for each reference object of the plurality of reference objects:
∘ causing the generative machine learning model (MLM) to generate a first synthetic representation (SR1) of the examination area of the reference object based on the first reference representation (RR1) and/or the second reference representation (RR2),
∘ generating a second synthetic representation (SR2) of the examination area of the reference object, wherein generating the second synthetic representation (SR2) comprises: subtracting the first reference representation (RR1) from the first synthetic representation (SR1),
∘ generating a third synthetic representation (SR3) of the examination area of the reference object, wherein generating the third synthetic representation (SR3) comprises: reducing the contrast of the second synthetic representation (SR2),
∘ generating a fourth synthetic representation (SR4) of the examination area of the reference object, wherein generating the fourth synthetic representation (SR4) comprises: subtracting the third synthetic representation (SR3) from the first synthetic representation (SR1) or adding the third synthetic representation (SR3) to the first reference representation (RR1),
∘ determining deviations between the fourth synthetic representation (SR4) and the second reference representation (RR2),
∘ reducing the deviations by modifying parameters of the generative machine learning model (MLM),
- providing a first representation (R1) and/or a second representation (R2) of the examination area of an examination object, wherein the first representation (R1) represents the examination area of the examination object without contrast agent, and the second representation (R2) represents the examination area of the examination object after application of the first amount of the contrast agent,
- causing the trained generative machine learning model (MLM^{t}) to generate a synthetic representation (SR) of the examination area of the examination object based on the first representation (R1) and/or the second representation (R2),
- outputting the synthetic representation (SR) of the examination area of the examination object and/or storing the synthetic representation (SR) of the examination area of the examination object in a data storage and/or transmitting the synthetic representation (SR) of the examination area of the examination object to a separate computer system.

13. A non-transitory computer readable storage medium having stored thereon a computer program that, when executed by a processing unit (20) of a computer system (1), cause the computer system (1) to perform the following steps:
- providing the trained generative machine learning model (MLM^{t}),
• wherein the trained generative machine learning model (MLM^{t}) was trained on training data (TD),
• wherein the training data (TD) comprised, for each reference object of a plurality of reference objects, a first reference representation (RR1) representing an examination area of the reference object without contrast agent, and a second reference representation (RR2) representing the examination area of the reference object after application of a first amount of a contrast agent,
• wherein training the generative machine learning model (MLM) comprised, for each reference object of the plurality of reference objects:
∘ causing the generative machine learning model (MLM) to generate a first synthetic representation (SR1) of the examination area of the reference object based on the first reference representation (RR1) and/or the second reference representation (RR2),
∘ generating a second synthetic representation (SR2) of the examination area of the reference object, wherein generating the second synthetic representation (SR2) comprises: subtracting the first reference representation (RR1) from the first synthetic representation (SR1),
∘ generating a third synthetic representation (SR3) of the examination area of the reference object, wherein generating the third synthetic representation (SR3) comprises: reducing the contrast of the second synthetic representation (SR2),
∘ generating a fourth synthetic representation (SR4) of the examination area of the reference object, wherein generating the fourth synthetic representation (SR4) comprises: subtracting the third synthetic representation (SR3) from the first synthetic representation (SR1) or adding the third synthetic representation (SR3) to the first reference representation (RR1),
∘ determining deviations between the fourth synthetic representation (SR4) and the second reference representation (RR2),
∘ reducing the deviations by modifying parameters of the generative machine learning model (MLM),
- providing a first representation (R1) and/or a second representation (R2) of the examination area of an examination object, wherein the first representation (R1) represents the examination area of the examination object without contrast agent, and the second representation (R2) represents the examination area of the examination object after application of the first amount of the contrast agent,
- causing the trained generative machine learning model (MLM^{t}) to generate a synthetic representation (SR) of the examination area of the examination object based on the first representation (R1) and/or the second representation (R2),
- outputting the synthetic representation (SR) of the examination area of the examination object and/or storing the synthetic representation (SR) of the examination area of the examination object in a data storage and/or transmitting the synthetic representation (SR) of the examination area of the examination object to a separate computer system.

14. Use of a contrast agent in an examination of an examination area of an examination object, the examination comprising:
- providing a trained generative machine learning model (MLM^{t}),
• wherein the trained generative machine learning model (MLM^{t}) was trained on training data (TD),
• wherein the training data (TD) comprised, for each reference object of a plurality of reference objects, a first reference representation (RR1) representing an examination area of the reference object without contrast agent, and a second reference representation (RR2) representing the examination area of the reference object after application of an amount of the contrast agent,
• wherein training the generative machine learning model (MLM) comprised, for each reference object of the plurality of reference objects:
∘ causing the generative machine learning model (MLM) to generate a first synthetic representation (SR1) of the examination area of the reference object based on the first reference representation (RR1) and/or the second reference representation (RR2),
∘ generating a second synthetic representation (SR2) of the examination area of the reference object, wherein generating the second synthetic representation (SR2) comprises: subtracting the first reference representation (RR1) from the first synthetic representation (SR1),
∘ generating a third synthetic representation (SR3) of the examination area of the reference object, wherein generating the third synthetic representation (SR3) comprises: reducing the contrast of the second synthetic representation (SR2),
∘ generating a fourth synthetic representation (SR4) of the examination area of the reference object, wherein generating the fourth synthetic representation (SR4) comprises: subtracting the third synthetic representation (SR3) from the first synthetic representation (SR1) or adding the third synthetic representation (SR3) to the first reference representation (RR1),
∘ determining deviations between the fourth synthetic representation (SR4) and the second reference representation (RR2),
∘ reducing the deviations by modifying parameters of the generative machine learning model (MLM),
- generating a first representation (R1) and/or a second representation (R2) of the examination area of an examination object, wherein the first representation (R1) represents the examination area of the examination object without contrast agent, and the second representation (R2) represents the examination area of the examination object after application of the amount of the contrast agent,
- causing the trained generative machine learning model (MLM^{t}) to generate a synthetic representation (SR) of the examination area of the examination object based on the first representation (R1) and/or the second representation (R2),
- outputting the synthetic representation (SR) of the examination area of the examination object and/or storing the synthetic representation (SR) of the examination area of the examination object in a data storage and/or transmitting the synthetic representation (SR) of the examination area of the examination object to a separate computer system.

15. Use of the contrast agent of claim 14, wherein the contrast agent comprises
- a Gd³⁺ complex of a compound of the formula (I) 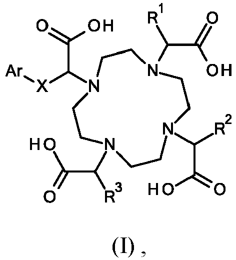 where
Ar is a group selected from 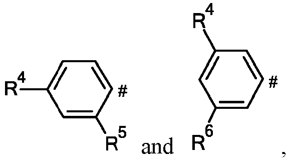
where # is the linkage to X,
X is a group selected from
CH₂, (CH₂)₂, (CH₂)₃, (CH₂)₄ and *-(CH₂)₂-O-CH₂-^{#} ,
where * is the linkage to Ar and ^{#} is the linkage to the acetic acid residue,
R¹, R² and R³ are each independently a hydrogen atom or a group selected from C₁-C₃ alkyl, -CH₂OH, -(CH₂)₂OH and -CH₂OCH₃,
R⁴ is a group selected from C₂-C₄ alkoxy, (H₃C-CH₂)-O-(CH₂)₂-O-, (H₃C-CH₂)-O-(CH₂)₂-O-(CH₂)₂-O- and (H₃C-CH₂)-O-(CH₂)₂-O-(CH₂)₂-O-(CH₂)₂-O-,
R⁵ is a hydrogen atom,
and
R⁶ is a hydrogen atom,
or a stereoisomer, tautomer, hydrate, solvate or salt thereof, or a mixture thereof, or
- a Gd³⁺ complex of a compound of the formula (II) 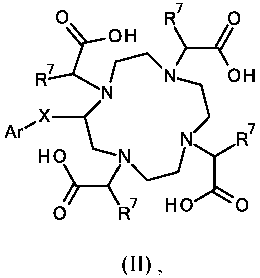 where
Ar is a group selected from 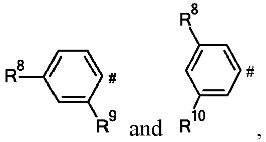
where # is the linkage to X,
X is a group selected from CH₂, (CH₂)₂, (CH₂)₃, (CH₂)₄ and *-(CH₂)₂-O-CH₂-^{#}, where * is the linkage to Ar and ^{#} is the linkage to the acetic acid residue,
R⁷ is a hydrogen atom or a group selected from C₁-C₃ alkyl, -CH₂OH,
-(CH₂)₂OH and -CH₂OCH₃;
R⁸ is a group selected from
C₂-C₄ alkoxy, (H₃C-CH₂O)-(CH₂)₂-O-, (H₃C-CH₂O)-(CH₂)₂-O-(CH₂)₂-O- and (H₃C-CH₂O)-(CH₂)₂-O-(CH₂)₂-O-(CH₂)₂-O-;
R⁹ and R¹⁰ independently represent a hydrogen atom;
or a stereoisomer, tautomer, hydrate, solvate or salt thereof, or a mixture thereof, or
the contrast agent comprises one of the following substances:
- gadolinium(III) 2-[4,7,10-tris(carboxymethyl)-1,4,7,10-tetrazacyclododec-1-yl]acetic acid,
- gadolinium(III) ethoxybenzyldiethylenetriaminepentaacetic acid,
- gadolinium(III) 2-[3,9-bis[1-carboxylato-4-(2,3-dihydroxypropylamino)-4-oxobutyl]-3,6,9,15-tetrazabicyclo[9.3.1]pentadeca-1(15),11,13-trien-6-yl]-5-(2,3-dihydroxypropylamino)-5-oxopentanoate,
- dihydrogen [(±)-4-carboxy-5,8,11-tris(carboxymethyl)-1-phenyl-2-oxa-5,8,11-triazatridecan-13-oato(5-)]gadolinate(2-),
- tetragadolinium [4,10-bis(carboxylatomethyl)-7-{3,6,12,15-tetraoxo-16-[4,7,10-tris(carboxylatomethyl)-1,4,7,10-tetraazacyclododecan-1-yl]-9,9-bis({[({2-[4,7,10-tris(carboxylatomethyl)-1,4,7,10-tetraazacyclododecan-1-yl]propanoyl}amino)acetyl]-amino}methyl)-4,7,11,14-tetraazahepta-decan-2-yl}-1,4,7,10-tetraazacyclododecan-1-yl]acetate,
- 2,2',2"-(10-{1-carboxy-2-[2-(4-ethoxyphenyl)ethoxy]ethyl}-1,4,7,10-tetraazacyclododecane-1,4,7-triyl)triacetate,
- gadolinium 2,2',2"-{10-[1-carboxy-2-{4-[2-(2-ethoxyethoxy)ethoxy]phenyl}ethyl]-1,4,7,10-tetraazacyclododecane-1,4,7-triyl}triacetate,
- gadolinium 2,2',2"-{10-[(1R)-1-carboxy-2-{4-[2-(2-ethoxyethoxy)ethoxy]phenyl}ethyl]-1,4,7,10-tetraazacyclododecane-1,4,7-triyl}triacetate,
- gadolinium (2S,2'S,2"S)-2,2',2"-{10-[(1S)-1-carboxy-4-{4-[2-(2-ethoxyethoxy)ethoxy] phenyl}butyl]-1,4,7,10-tetraazacyclododecane-1,4,7-triyl}tris(3-hydroxypropanoate)
- gadolinium 2,2',2"-{10-[(1S)-4-(4-butoxyphenyl)-1-carboxybutyl]-1,4,7,10-tetraazacyclododecane-1,4,7-triyl}triacetate,
- gadolinium(III) 5,8-bis(carboxylatomethyl)-2-[2-(methylamino)-2-oxoethyl]-10-oxo-2,5,8,11-tetraazadodecane-1-carboxylate hydrate
- gadolinium(III) 2-[4-(2-hydroxypropyl)-7,10-bis(2-oxido-2-oxoethyl)-1,4,7,10-tetrazacyclododec-1-yl]acetate,
- gadolinium(III) 2,2',2"-(10-((2R,3S)-1,3,4-trihydroxybutan-2-yl)-1,4,7,10-tetraazacyclododecane-1,4,7-triyl)triacetate,
- gadolinium-2,2',2"-{(2S)-10-(carboxymethyl)-2-[4-(2-ethoxyethoxy)benzyl]-1,4,7,10-tetraazacyclododecane-1,4,7-triyl}triacetate,
- gadolinium-2,2',2"-[10-(carboxymethyl)-2-(4-ethoxybenzyl)-1,4,7,10-tetraazacyclododecane-1,4,7-triyl]triacetate.

16. Kit comprising a computer program according to claim 13 and a contrast agent, wherein the contrast agent comprises
- a Gd³⁺ complex of a compound of the formula (I) 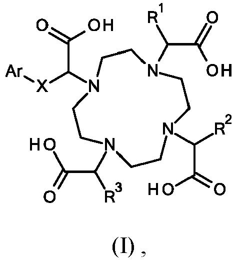 where
Ar is a group selected from 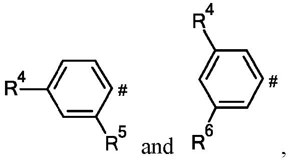
where # is the linkage to X,
X is a group selected from
CH₂, (CH₂)₂, (CH₂)₃, (CH₂)₄ and *-(CH₂)₂-O-CH₂-^{#} ,
where * is the linkage to Ar and ^{#} is the linkage to the acetic acid residue,
R¹, R² and R³ are each independently a hydrogen atom or a group selected from C₁-C₃ alkyl, -CH₂OH, -(CH₂)₂OH and -CH₂OCH₃,
R⁴ is a group selected from C₂-C₄ alkoxy, (H₃C-CH₂)-O-(CH₂)₂-O-, (H₃C-CH₂)-O-(CH₂)₂-O-(CH₂)₂-O- and (H₃C-CH₂)-O-(CH₂)₂-O-(CH₂)₂-O-(CH₂)₂-O-,
R⁵ is a hydrogen atom,
and
R⁶ is a hydrogen atom,
or a stereoisomer, tautomer, hydrate, solvate or salt thereof, or a mixture thereof, or
- a Gd³⁺ complex of a compound of the formula (II) 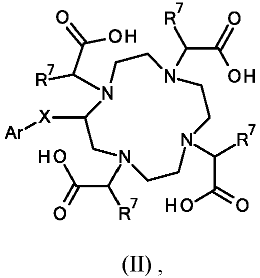 where
Ar is a group selected from 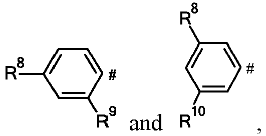
where # is the linkage to X,
X is a group selected from CH₂, (CH₂)₂, (CH₂)₃, (CH₂)₄ and *-(CH₂)₂-O-CH₂-^{#}, where * is the linkage to Ar and ^{#} is the linkage to the acetic acid residue,
R⁷ is a hydrogen atom or a group selected from C₁-C₃ alkyl, -CH₂OH, -(CH₂)₂OH and -CH₂OCH₃;
R⁸ is a group selected from
C₂-C₄ alkoxy, (H₃C-CH₂O)-(CH₂)₂-O-, (H₃C-CH₂O)-(CH₂)₂-O-(CH₂)₂-O- and (H₃C-CH₂O)-(CH₂)₂-O-(CH₂)₂-O-(CH₂)₂-O-;
R⁹ and R¹⁰ independently represent a hydrogen atom;
or a stereoisomer, tautomer, hydrate, solvate or salt thereof, or a mixture thereof, or
the contrast agent comprises one of the following substances:
- gadolinium(III) 2-[4,7,10-tris(carboxymethyl)-1,4,7,10-tetrazacyclododec-1-yl]acetic acid,
- gadolinium(III) ethoxybenzyldiethylenetriaminepentaacetic acid,
- gadolinium(III) 2-[3,9-bis[1-carboxylato-4-(2,3-dihydroxypropylamino)-4-oxobutyl]-3,6,9,15-tetrazabicyclo[9.3.1]pentadeca-1(15),11,13-trien-6-yl]-5-(2,3-dihydroxypropylamino)-5-oxopentanoate,
- dihydrogen [(±)-4-carboxy-5,8,11-tris(carboxymethyl)-1-phenyl-2-oxa-5,8,11-triazatridecan-13-oato(5-)]gadolinate(2-),
- tetragadolinium [4,10-bis(carboxylatomethyl)-7-{3,6,12,15-tetraoxo-16-[4,7,10-tris(carboxylatomethyl)-1,4,7,10-tetraazacyclododecan-1-yl]-9,9-bis({[({2-[4,7,10-tris(carboxylatomethyl)-1,4,7,10-tetraazacyclododecan-1-yl]propanoyl}amino)acetyl]-amino}methyl)-4,7,11,14-tetraazahepta-decan-2-yl}-1,4,7,10-tetraazacyclododecan-1-yl]acetate,
- 2,2',2"-(10-{1-carboxy-2-[2-(4-ethoxyphenyl)ethoxy]ethyl}-1,4,7,10-tetraazacyclododecane-1,4,7-triyl)triacetate,
- gadolinium 2,2',2"-{10-[1-carboxy-2-{4-[2-(2-ethoxyethoxy)ethoxy]phenyl}ethyl]-1,4,7,10-tetraazacyclododecane-1,4,7-triyl}triacetate,
- gadolinium 2,2',2"-{10-[(1R)-1-carboxy-2-{4-[2-(2-ethoxyethoxy)ethoxy]phenyl}ethyl]-1,4,7,10-tetraazacyclododecane-1,4,7-triyl}triacetate,
- gadolinium (2S,2'S,2"S)-2,2',2"-{10-[(1S)-1-carboxy-4-{4-[2-(2-ethoxyethoxy)ethoxy] phenyl}butyl]-1,4,7,10-tetraazacyclododecane-1,4,7-triyl}tris(3-hydroxypropanoate)
- gadolinium 2,2',2"-{10-[(1S)-4-(4-butoxyphenyl)-1-carboxybutyl]-1,4,7,10-tetraazacyclododecane-1,4,7-triyl}triacetate,
- gadolinium(III) 5,8-bis(carboxylatomethyl)-2-[2-(methylamino)-2-oxoethyl]-10-oxo-2,5,8,11-tetraazadodecane-1-carboxylate hydrate
- gadolinium(III) 2-[4-(2-hydroxypropyl)-7,10-bis(2-oxido-2-oxoethyl)-1,4,7,10-tetrazacyclododec-1-yl]acetate,
- gadolinium(III) 2,2',2"-(10-((2R,3S)-1,3,4-trihydroxybutan-2-yl)-1,4,7,10-tetraazacyclododecane-1,4,7-triyl)triacetate,
- gadolinium-2,2',2"-{(2S)-10-(carboxymethyl)-2-[4-(2-ethoxyethoxy)benzyl]-1,4,7,10-tetraazacyclododecane-1,4,7-triyl}triacetate,
- gadolinium-2,2',2"-[10-(carboxymethyl)-2-(4-ethoxybenzyl)-1,4,7,10-tetraazacyclododecane-1,4,7-triyl]triacetate.

## Patentansprüche

1. Computerimplementiertes Verfahren zum Trainieren eines generativen Modells für maschinelles Lernen (MLM), wobei das Verfahren Folgendes umfasst:
- Bereitstellen des generativen Modells für maschinelles Lernen (MLM),
- Bereitstellen von Trainingsdaten (TD), wobei die Trainingsdaten (TD) für jedes Referenzobjekt einer Mehrzahl von Referenzobjekten eine erste Referenzdarstellung (RR1), die einen Untersuchungsbereich des Referenzobjekts ohne Kontrastmittel darstellt, und eine zweite Referenzdarstellung (RR2) umfassen, die den Untersuchungsbereich des Referenzobjekts nach Anwendung einer ersten Menge eines Kontrastmittels darstellt,
- Trainieren des generativen Modells für maschinelles Lernen (MLM), wobei Trainieren des generativen Modells für maschinelles Lernen (MLM) für jedes Referenzobjekt der Mehrzahl von Referenzobjekten Folgendes umfasst:
• Veranlassen des generativen Modells für maschinelles Lernen (MLM), eine erste synthetische Darstellung (SR1) des Untersuchungsbereichs des Referenzobjekts basierend auf der ersten Referenzdarstellung (RR1) und/oder der zweiten Referenzdarstellung (RR2) zu erzeugen,
• Erzeugen einer zweiten synthetischen Darstellung (SR2) des Untersuchungsbereichs des Referenzobjekts, wobei das Erzeugen der zweiten synthetischen Darstellung (SR2) Folgendes umfasst: Subtrahieren der ersten Referenzdarstellung (RR1) von der ersten synthetischen Darstellung (SR1),
• Erzeugen einer dritten synthetischen Darstellung (SR3) des Untersuchungsbereichs des Referenzobjekts, wobei das Erzeugen der dritten synthetischen Darstellung (SR3) Folgendes umfasst: Reduzieren des Kontrasts der zweiten synthetischen Darstellung (SR2),
• Erzeugen einer vierten synthetischen Darstellung (SR4) des Untersuchungsbereichs des Referenzobjekts, wobei das Erzeugen der vierten synthetischen Darstellung (SR4) Folgendes umfasst: Subtrahieren der dritten synthetischen Darstellung (SR3) von der ersten synthetischen Darstellung (SR1) oder Hinzufügen der dritten synthetischen Darstellung (SR3) zu der ersten Referenzdarstellung (RR1),
• Bestimmen von Abweichungen zwischen der vierten synthetischen Darstellung (SR4) und der zweiten Referenzdarstellung (RR2),
• Reduzieren der Abweichungen durch Modifizieren von Parametern des generativen Modells für maschinelles Lernen (MLM),
- Speichern des trainierten generativen Modells für maschinelles Lernen (MLM^{t}) und/oder Übertragen des trainierten generativen Modells für maschinelles Lernen (MLM^{t}) an ein anderes Computersystem und/oder Verwenden des trainierten generativen Modells für maschinelles Lernen (MLM^{t}), um eine synthetische Darstellung (SR) des Untersuchungsbereichs eines Untersuchungsobjekts zu erzeugen.

2. Computerimplementiertes Verfahren zum Erzeugen einer synthetischen Darstellung (SR) unter Verwendung eines trainierten generativen Modells für maschinelles Lernen (MLM^{t}), wobei das Verfahren Folgendes umfasst:
- Bereitstellen des trainierten generativen Modells für maschinelles Lernen (MLM^{t}),
• wobei das trainierte generative Modell für maschinelles Lernen (MLM^{t}) mit Trainingsdaten (TD) trainiert wurde,
• wobei die Trainingsdaten (TD) für jedes Referenzobjekt einer Mehrzahl von Referenzobjekten eine erste Referenzdarstellung (RR1), die einen Untersuchungsbereich des Referenzobjekts ohne Kontrastmittel darstellt, und eine zweite Referenzdarstellung (RR2) umfassten, die den Untersuchungsbereich des Referenzobjekts nach Anwendung einer ersten Menge eines Kontrastmittels darstellt,
• wobei das Trainieren des generativen Modells für maschinelles Lernen (MLM) für jedes Referenzobjekt der Mehrzahl von Referenzobjekten Folgendes umfasste:
∘ Veranlassen des generativen Modells für maschinelles Lernen (MLM), eine erste synthetische Darstellung (SR1) des Untersuchungsbereichs des Referenzobjekts basierend auf der ersten Referenzdarstellung (RR1) und/oder der zweiten Referenzdarstellung (RR2) zu erzeugen,
∘ Erzeugen einer zweiten synthetischen Darstellung (SR2) des Untersuchungsbereichs des Referenzobjekts, wobei das Erzeugen der zweiten synthetischen Darstellung (SR2) Folgendes umfasst: Subtrahieren der ersten Referenzdarstellung (RR1) von der ersten synthetischen Darstellung (SR1),
∘ Erzeugen einer dritten synthetischen Darstellung (SR3) des Untersuchungsbereichs des Referenzobjekts, wobei das Erzeugen der dritten synthetischen Darstellung (SR3) Folgendes umfasst: Reduzieren des Kontrasts der zweiten synthetischen Darstellung (SR2),
∘ Erzeugen einer vierten synthetischen Darstellung (SR4) des Untersuchungsbereichs des Referenzobjekts, wobei das Erzeugen der vierten synthetischen Darstellung (SR4) Folgendes umfasst: Subtrahieren der dritten synthetischen Darstellung (SR3) von der ersten synthetischen Darstellung (SR1) oder Hinzufügen der dritten synthetischen Darstellung (SR3) zu der ersten Referenzdarstellung (RR1),
∘ Bestimmen von Abweichungen zwischen der vierten synthetischen Darstellung (SR4) und der zweiten Referenzdarstellung (RR2),
∘ Reduzieren der Abweichungen durch Modifizieren von Parametern des generativen Modells für maschinelles Lernen (MLM),
- Bereitstellen einer ersten Darstellung (R1) und/oder einer zweiten Darstellung (R2) des Untersuchungsbereichs eines Untersuchungsobjekts, wobei die erste Darstellung (R1) den Untersuchungsbereich des Untersuchungsobjekts ohne Kontrastmittel darstellt und die zweite Darstellung (R2) den Untersuchungsbereich des Untersuchungsobjekts nach Anwendung der ersten Menge des Kontrastmittels darstellt,
- Veranlassen des trainierten generativen Modells für maschinelles Lernen (MLM^{t}), eine synthetische Darstellung (SR) des Untersuchungsbereichs des Untersuchungsobjekts basierend auf der ersten Darstellung (R1) und/oder der zweiten Darstellung (R2) zu erzeugen,
- Ausgeben der synthetischen Darstellung (SR) des Untersuchungsbereichs des Untersuchungsobjekts und/oder Speichern der synthetischen Darstellung (SR) des Untersuchungsbereichs des Untersuchungsobjekts in einem Datenspeicher und/oder Übertragen der synthetischen Darstellung (SR) des Untersuchungsbereichs des Untersuchungsobjekts an ein separates Computersystem.

3. Verfahren nach Anspruch 1 oder 2, wobei jedes Referenzobjekt ein Lebewesen, z. B. ein Säuger, z. B. ein Mensch, ist und das Untersuchungsobjekt ein Lebewesen, z. B. ein Säuger, z. B. ein Mensch, ist.

4. Verfahren nach einem der Ansprüche 1 bis 3, wobei der Untersuchungsbereich eine Leber, eine Niere, ein Herz, eine Lunge, ein Gehirn, ein(en) Magen, eine Blase, eine Prostata, ein(en) Darm, eine Brust, eine Schilddrüse, eine Bauchspeicheldrüse, eine Gebärmutter oder ein(en) Teil davon eines Säugers, z. B. eines Menschen, ist oder umfasst.

5. Verfahren nach einem der Ansprüche 1 bis 4, wobei jede Darstellung eine radiologische Darstellung ist.

6. Verfahren nach einem der Ansprüche 1 bis 5, wobei die synthetische Darstellung (SR) des Untersuchungsbereichs des Untersuchungsobjekts den Untersuchungsbereich des Untersuchungsobjekts nach Anwendung einer zweiten Menge des Kontrastmittels darstellt, wobei die zweite Menge größer als die erste Menge ist.

7. Verfahren nach einem der Ansprüche 1 bis 6, wobei das Reduzieren des Kontrasts der zweiten synthetischen Darstellung (SR2) Folgendes umfasst: lineare oder nichtlineare Dämpfung von Grauwerten oder Farbwerten von Bildelementen der zweiten synthetischen Darstellung (SR2) .

8. Verfahren nach einem der Ansprüche 1 bis 7, wobei das Reduzieren des Kontrasts der zweiten synthetischen Darstellung (SR2) Folgendes umfasst: Multiplizieren von Grauwerten oder Farbwerten von Bildelementen der zweiten synthetischen Darstellung (SR2) mit einem Dämpfungsfaktor, wobei der Dämpfungsfaktor größer als null und kleiner als 1 ist.

9. Verfahren nach einem der Ansprüche 1 bis 8, wobei das generative Modell für maschinelles Lernen (MLM) eines oder mehrere von Folgenden ist oder umfasst: ein künstliches neuronales Netzwerk, ein neuronales Faltungsnetzwerk, ein Variations-Autoencoder, ein generatives kontradiktorisches Netzwerk, ein Transformator, ein Diffusionsnetzwerk.

10. Verfahren nach einem der Ansprüche 1 bis 10, wobei der Untersuchungsbereich eine menschliche Leber ist oder umfasst oder Teil einer menschlichen Leber ist und das Kontrastmittel ein hepatobiliäres Kontrastmittel, z. B. ein hepatobiliäres MRT-Kontrastmittel, ist.

11. Verfahren nach einem der Ansprüche 1 bis 11, wobei die Erzeugung der ersten synthetischen Darstellung (SR1) des Untersuchungsbereichs des Referenzobjekts auf der ersten Referenzdarstellung (RR1) und der zweiten Referenzdarstellung (RR2) basiert und die Erzeugung der synthetischen Darstellung (SR) des Untersuchungsbereichs des Untersuchungsobjekts auf der ersten Darstellung (R1) und der zweiten Darstellung (R2) basiert.

12. Computersystem (1), umfassend:
eine Verarbeitungseinheit (20); und
einen Speicher (50), der ein Computerprogramm (60) speichert, das dazu ausgelegt ist, bei Ausführung durch die Verarbeitungseinheit (20) eine Operation auszuführen, wobei die Operation Folgendes umfasst:
- Bereitstellen des trainierten generativen Modells für maschinelles Lernen (MLM^{t}),
• wobei das trainierte generative Modell für maschinelles Lernen (MLM^{t}) mit Trainingsdaten (TD) trainiert wurde,
• wobei die Trainingsdaten (TD) für jedes Referenzobjekt einer Mehrzahl von Referenzobjekten eine erste Referenzdarstellung (RR1), die einen Untersuchungsbereich des Referenzobjekts ohne Kontrastmittel darstellt, und eine zweite Referenzdarstellung (RR2) umfassten, die den Untersuchungsbereich des Referenzobjekts nach Anwendung einer ersten Menge eines Kontrastmittels darstellt,
• wobei das Trainieren des generativen Modells für maschinelles Lernen (MLM) für jedes Referenzobjekt der Mehrzahl von Referenzobjekten Folgendes umfasste:
∘ Veranlassen des generativen Modells für maschinelles Lernen (MLM), eine erste synthetische Darstellung (SR1) des Untersuchungsbereichs des Referenzobjekts basierend auf der ersten Referenzdarstellung (RR1) und/oder der zweiten Referenzdarstellung (RR2) zu erzeugen,
∘ Erzeugen einer zweiten synthetischen Darstellung (SR2) des Untersuchungsbereichs des Referenzobjekts, wobei das Erzeugen der zweiten synthetischen Darstellung (SR2) Folgendes umfasst: Subtrahieren der ersten Referenzdarstellung (RR1) von der ersten synthetischen Darstellung (SR1),
∘ Erzeugen einer dritten synthetischen Darstellung (SR3) des Untersuchungsbereichs des Referenzobjekts, wobei das Erzeugen der dritten synthetischen Darstellung (SR3) Folgendes umfasst: Reduzieren des Kontrasts der zweiten synthetischen Darstellung (SR2),
∘ Erzeugen einer vierten synthetischen Darstellung (SR4) des Untersuchungsbereichs des Referenzobjekts, wobei das Erzeugen der vierten synthetischen Darstellung (SR4) Folgendes umfasst: Subtrahieren der dritten synthetischen Darstellung (SR3) von der ersten synthetischen Darstellung (SR1) oder Hinzufügen der dritten synthetischen Darstellung (SR3) zu der ersten Referenzdarstellung (RR1),
∘ Bestimmen von Abweichungen zwischen der vierten synthetischen Darstellung (SR4) und der zweiten Referenzdarstellung (RR2),
∘ Reduzieren der Abweichungen durch Modifizieren von Parametern des generativen Modells für maschinelles Lernen (MLM),
- Bereitstellen einer ersten Darstellung (R1) und/oder einer zweiten Darstellung (R2) des Untersuchungsbereichs eines Untersuchungsobjekts, wobei die erste Darstellung (R1) den Untersuchungsbereich des Untersuchungsobjekts ohne Kontrastmittel darstellt und die zweite Darstellung (R2) den Untersuchungsbereich des Untersuchungsobjekts nach Anwendung der ersten Menge des Kontrastmittels darstellt,
- Veranlassen des trainierten generativen Modells für maschinelles Lernen (MLM^{t}), eine synthetische Darstellung (SR) des Untersuchungsbereichs des Untersuchungsobjekts basierend auf der ersten Darstellung (R1) und/oder der zweiten Darstellung (R2) zu erzeugen,
- Ausgeben der synthetischen Darstellung (SR) des Untersuchungsbereichs des Untersuchungsobjekts und/oder Speichern der synthetischen Darstellung (SR) des Untersuchungsbereichs des Untersuchungsobjekts in einem Datenspeicher und/oder Übertragen der synthetischen Darstellung (SR) des Untersuchungsbereichs des Untersuchungsobjekts an ein separates Computersystem.

13. Nichtflüchtiges, computerlesbares Speichermedium mit einem darauf gespeicherten Computerprogramm, das bei Ausführung durch eine Verarbeitungseinheit (20) eines Computersystems (1) das Computersystem (1) veranlasst, die folgenden Schritte auszuführen:
- Bereitstellen des trainierten generativen Modells für maschinelles Lernen (MLM^{t}),
• wobei das trainierte generative Modell für maschinelles Lernen (MLM^{t}) mit Trainingsdaten (TD) trainiert wurde,
• wobei die Trainingsdaten (TD) für jedes Referenzobjekt einer Mehrzahl von Referenzobjekten eine erste Referenzdarstellung (RR1), die einen Untersuchungsbereich des Referenzobjekts ohne Kontrastmittel darstellt, und eine zweite Referenzdarstellung (RR2) umfassten, die den Untersuchungsbereich des Referenzobjekts nach Anwendung einer ersten Menge eines Kontrastmittels darstellt,
• wobei das Trainieren des generativen Modells für maschinelles Lernen (MLM) für jedes Referenzobjekt der Mehrzahl von Referenzobjekten Folgendes umfasste:
∘ Veranlassen des generativen Modells für maschinelles Lernen (MLM), eine erste synthetische Darstellung (SR1) des Untersuchungsbereichs des Referenzobjekts basierend auf der ersten Referenzdarstellung (RR1) und/oder der zweiten Referenzdarstellung (RR2) zu erzeugen,
∘ Erzeugen einer zweiten synthetischen Darstellung (SR2) des Untersuchungsbereichs des Referenzobjekts, wobei das Erzeugen der zweiten synthetischen Darstellung (SR2) Folgendes umfasst: Subtrahieren der ersten Referenzdarstellung (RR1) von der ersten synthetischen Darstellung (SR1),
∘ Erzeugen einer dritten synthetischen Darstellung (SR3) des Untersuchungsbereichs des Referenzobjekts, wobei das Erzeugen der dritten synthetischen Darstellung (SR3) Folgendes umfasst: Reduzieren des Kontrasts der zweiten synthetischen Darstellung (SR2),
∘ Erzeugen einer vierten synthetischen Darstellung (SR4) des Untersuchungsbereichs des Referenzobjekts, wobei das Erzeugen der vierten synthetischen Darstellung (SR4) Folgendes umfasst: Subtrahieren der dritten synthetischen Darstellung (SR3) von der ersten synthetischen Darstellung (SR1) oder Hinzufügen der dritten synthetischen Darstellung (SR3) zu der ersten Referenzdarstellung (RR1),
∘ Bestimmen von Abweichungen zwischen der vierten synthetischen Darstellung (SR4) und der zweiten Referenzdarstellung (RR2),
∘ Reduzieren der Abweichungen durch Modifizieren von Parametern des generativen Modells für maschinelles Lernen (MLM),
- Bereitstellen einer ersten Darstellung (R1) und/oder einer zweiten Darstellung (R2) des Untersuchungsbereichs eines Untersuchungsobjekts, wobei die erste Darstellung (R1) den Untersuchungsbereich des Untersuchungsobjekts ohne Kontrastmittel darstellt und die zweite Darstellung (R2) den Untersuchungsbereich des Untersuchungsobjekts nach Anwendung der ersten Menge des Kontrastmittels darstellt,
- Veranlassen des trainierten generativen Modells für maschinelles Lernen (MLM^{t}), eine synthetische Darstellung (SR) des Untersuchungsbereichs des Untersuchungsobjekts basierend auf der ersten Darstellung (R1) und/oder der zweiten Darstellung (R2) zu erzeugen,
- Ausgeben der synthetischen Darstellung (SR) des Untersuchungsbereichs des Untersuchungsobjekts und/oder Speichern der synthetischen Darstellung (SR) des Untersuchungsbereichs des Untersuchungsobjekts in einem Datenspeicher und/oder Übertragen der synthetischen Darstellung (SR) des Untersuchungsbereichs des Untersuchungsobjekts an ein separates Computersystem.

14. Verwendung eines Kontrastmittels bei einer Untersuchung eines Untersuchungsbereichs eines Untersuchungsobjekts, wobei die Untersuchung Folgendes umfasst:
- Bereitstellen eines trainierten generativen Modells für maschinelles Lernen (MLM^{t}),
• wobei das trainierte generative Modell für maschinelles Lernen (MLM^{t}) mit Trainingsdaten (TD) trainiert wurde,
• wobei die Trainingsdaten (TD) für jedes Referenzobjekt einer Mehrzahl von Referenzobjekten eine erste Referenzdarstellung (RR1), die einen Untersuchungsbereich des Referenzobjekts ohne Kontrastmittel darstellt, und eine zweite Referenzdarstellung (RR2) umfassten, die den Untersuchungsbereich des Referenzobjekts nach Anwendung einer ersten Menge eines Kontrastmittels darstellt,
• wobei das Trainieren des generativen Modells für maschinelles Lernen (MLM) für jedes Referenzobjekt der Mehrzahl von Referenzobjekten Folgendes umfasste:
∘ Veranlassen des generativen Modells für maschinelles Lernen (MLM), eine erste synthetische Darstellung (SR1) des Untersuchungsbereichs des Referenzobjekts basierend auf der ersten Referenzdarstellung (RR1) und/oder der zweiten Referenzdarstellung (RR2) zu erzeugen,
∘ Erzeugen einer zweiten synthetischen Darstellung (SR2) des Untersuchungsbereichs des Referenzobjekts, wobei das Erzeugen der zweiten synthetischen Darstellung (SR2) Folgendes umfasst: Subtrahieren der ersten Referenzdarstellung (RR1) von der ersten synthetischen Darstellung (SR1),
∘ Erzeugen einer dritten synthetischen Darstellung (SR3) des Untersuchungsbereichs des Referenzobjekts, wobei das Erzeugen der dritten synthetischen Darstellung (SR3) Folgendes umfasst: Reduzieren des Kontrasts der zweiten synthetischen Darstellung (SR2),
∘ Erzeugen einer vierten synthetischen Darstellung (SR4) des Untersuchungsbereichs des Referenzobjekts, wobei das Erzeugen der vierten synthetischen Darstellung (SR4) Folgendes umfasst: Subtrahieren der dritten synthetischen Darstellung (SR3) von der ersten synthetischen Darstellung (SR1) oder Hinzufügen der dritten synthetischen Darstellung (SR3) zu der ersten Referenzdarstellung (RR1),
∘ Bestimmen von Abweichungen zwischen der vierten synthetischen Darstellung (SR4) und der zweiten Referenzdarstellung (RR2),
∘ Reduzieren der Abweichungen durch Modifizieren von Parametern des generativen Modells für maschinelles Lernen (MLM),
- Erzeugen einer ersten Darstellung (R1) und/oder einer zweiten Darstellung (R2) des Untersuchungsbereichs eines Untersuchungsobjekts, wobei die erste Darstellung (R1) den Untersuchungsbereich des Untersuchungsobjekts ohne Kontrastmittel darstellt und die zweite Darstellung (R2) den Untersuchungsbereich des Untersuchungsobjekts nach Anwendung der ersten Menge des Kontrastmittels darstellt,
- Veranlassen des trainierten generativen Modells für maschinelles Lernen (MLM^{t}), eine synthetische Darstellung (SR) des Untersuchungsbereichs des Untersuchungsobjekts basierend auf der ersten Darstellung (R1) und/oder der zweiten Darstellung (R2) zu erzeugen,
- Ausgeben der synthetischen Darstellung (SR) des Untersuchungsbereichs des Untersuchungsobjekts und/oder Speichern der synthetischen Darstellung (SR) des Untersuchungsbereichs des Untersuchungsobjekts in einem Datenspeicher und/oder Übertragen der synthetischen Darstellung (SR) des Untersuchungsbereichs des Untersuchungsobjekts an ein separates Computersystem.

15. Verwendung des Kontrastmittels nach Anspruch 14, wobei das Kontrastmittel Folgendes umfasst:
- einen Gd³⁺⁻-Komplex einer Verbindung der folgenden Formel (I): 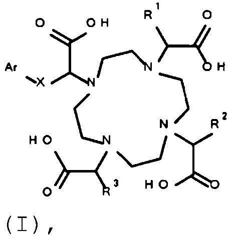 wobei
Ar für eine Gruppe steht, die ausgewählt ist aus 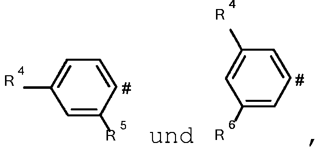
wobei # die Bindung an X ist,
X für eine Gruppe steht, die ausgewählt ist aus CH₂, (CH₂)₂, (CH₂)₃, (CH₂)₄ und *- (CH₂)₂-O-CH₂-^{#},
wobei * die Bindung an Ar ist und ^{#} die Bindung an den Essigsäurerest ist,
R¹, R² und R³ jeweils unabhängig für ein Wasserstoffatom oder eine Gruppe stehen, die ausgewählt ist aus C₁-C₃-Alkyl, -CH₂OH, - (CH₂)₂OH und -CH₂OCH₃,
R⁴ für eine Gruppe steht, die ausgewählt ist aus C₂-C₄-Alkoxy, (H₃C-CH₂)-O- (CH₂)₂-O-, (H₃C-CH₂)-O- (CH₂)₂-O- (CH₂)₂-O- und (H₃C-CH₂)-O- (CH₂)₂-O-(CH₂)₂-O-(CH₂)₂-O-,
R⁵ für ein Wasserstoffatom steht
und
R⁶ für ein Wasserstoffatom steht
oder ein Stereoisomer, ein Tautomer, ein Hydrat, ein Solvat oder ein Salz davon oder ein Gemisch davon, oder
- einen Gd³⁺⁻-Komplex einer Verbindung der folgenden Formel (II): 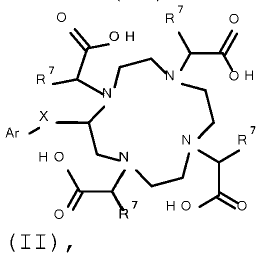 wobei
Ar für eine Gruppe steht, die ausgewählt ist aus 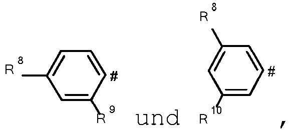
wobei # die Bindung an X ist,
X eine Gruppe ist, die ausgewählt ist aus CH₂, (CH₂)₂, (CH₂)₃, (CH₂)₄ und *-(CH₂)₂-O-CH₂-^{#}, wobei * die Bindung an Ar ist und ^{#} die Bindung an den Essigsäurerest ist,
R² für ein Wasserstoffatom oder eine Gruppe, die aus C₁-C₃-Alkyl, -CH₂OH, -(CH₂)₂OH und -CH₂OCH₃ ausgewählt ist, steht,
R⁸ für eine Gruppe steht, die ausgewählt ist aus C₂-C₄-Alkoxy, (H₃C-CH₂O)-(CH₂)₂-O-, (H₃C-CH₂O)-(CH₂)₂-O-(CH₂)₂-O- und (H₃C-CH₂O)- (CH₂)₂-O-(CH₂)₂-O-(CH₂)₂-O-,
R⁹ und R¹⁰ unabhängig voneinander ein Wasserstoffatom darstellen;
oder ein Stereoisomer, ein Tautomer, ein Hydrat, ein Solvat oder ein Salz davon oder ein Gemisch davon, oder das Kontrastmittel eine der folgenden Substanzen umfasst:
- Gadolinium(III)-2-[4,7,10-Tris(carboxymethyl)-1,4,7,10-tetrazacyclododec-1-yl]essigsäure,
- Gadolinium(III)-Ethoxybenzyldiethylentriaminpentaessigsäure,
- Gadolinium(III)-2-[3,9-Bis[1-carboxylato-4-(2,3-dihydroxypropylamino)-4-oxobutyl]-3,6,9,15-tetrazabicyclo[9.3.1]pentadeca-1(15),11,13-trien-6-yl]-5-(2,3-dihydroxypropylamino)-5-oxopentanoat,
- Dihydrogen-[(±)-4-Carboxy-5,8,11-tris(carboxymethyl)-1-phenyl-2-oxa-5,8,11-triazatridecan-13-oato(5-)]gadolinat(2-),
- Tetragadolinium-[4,10-Bis(carboxylatomethyl)-7-{3,6,12,15-tetraoxo-16-[4,7,10-tris(carboxylatomethyl)-1,4,7,10-tetraazacyclododecan-1-yl]-9,9-bis({[({2-[4,7,10-tris(carboxylatomethyl)-1,4,7,10-tetraazacyclododecan-1-yl]propanoyl}amino)acetyl]-amino}methyl)-4,7,11,14-tetraazahepta-decan-2-yl}-1,4,7,10-tetraazacyclododecan-1-yl]acetat,
- 2,2',2"-(10-{1-Carboxy-2-[2-(4-ethoxyphenyl)ethoxy]ethyl}-1,4,7,10-tetraazacyclododecan-1,4,7-triyl)triacetat,
- Gadolinium-2,2',2"-{10-[1-Carboxy-2-{4-[2-(2-ethoxyethoxy)ethoxy]phenyl}ethyl]-1,4,7,10-tetraazacyclododecan-1,4,7-triyl}triacetat,
- Gadolinium-2,2',2"-{10-[(1R)-1-Carboxy-2-{4-[2-(2-ethoxyethoxy)ethoxy]phenyl}ethyl]-1,4,7,10-tetraazacyclododecan-1,4,7-triyl}triacetat,
- Gadolinium-(2S,2'S,2"S)-2,2',2"-{10-[(1S)-1-Carboxy-4-{4-[2-(2-ethoxyethoxy)ethoxy]phenyl}butyl]-1,4,7,10-tetraazacyclododecan-1,4,7-triyl}tris(3-hydroxypropanoat),
- Gadolinium-2,2',2"-{10-[(1S)-4-(4-Butoxyphenyl)-1-carboxybutyl]-1,4,7,10-tetraazacyclododecan-1,4,7-triyl}triacetat,
- Gadolinium(III)-5,8-Bis(carboxylatomethyl)-2-[2-(methylamino)-2-oxoethyl]-10-oxo-2,5,8,11-tetraazadodecan-1-carboxylathydrat,
- Gadolinium(III)-2-[4-(2-Hydroxypropyl)-7,10-bis(2-oxido-2-oxoethyl)-1,4,7,10-tetrazacyclododec-1-yl]acetat,
- Gadolinium(III)-2,2',2"-(10-((2R,3S)-1,3,4-Trihydroxybutan-2-yl)-1,4,7,10-tetraazacyclododecan-1,4,7-triyl)triacetat,
- Gadolinium-2,2',2"-{(2S)-10-(carboxymethyl)-2-[4-(2-ethoxyethoxy)benzyl]-1,4,7,10-tetraazacyclododecan-1,4,7-triyl}triacetat,
- Gadolinium-2,2',2"-[10-(carboxymethyl)-2-(4-ethoxybenzyl)-1,4,7,10-tetraazacyclododecan-1,4,7-triyl]triacetat.

16. Kit, umfassend ein Computerprogramm nach Anspruch 13 und ein Kontrastmittel, wobei das Kontrastmittel Folgendes umfasst:
- einen Gd³⁺⁻-Komplex einer Verbindung der folgenden Formel (I): 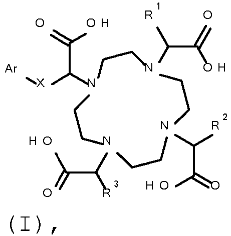 wobei
Ar für eine Gruppe steht, die ausgewählt ist aus 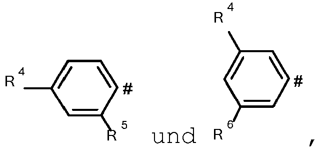
wobei # die Bindung an X ist,
X für eine Gruppe steht, die ausgewählt ist aus CH₂, (CH₂)₂, (CH₂)₃, (CH₂)₄ und *-(CH₂)₂-O-CH₂-^{#},
wobei * die Bindung an Ar ist und ^{#} die Bindung an den Essigsäurerest ist,
R¹, R² und R³ jeweils unabhängig für ein Wasserstoffatom oder eine Gruppe stehen, die ausgewählt ist aus C₁-C₃-Alkyl, -CH₂OH, -(CH₂)₂OH und -CH₂OCH₃,
R⁴ für eine Gruppe steht, die ausgewählt ist aus C₂-C₄-Alkoxy, (H₃C-CH₂)-O-(CH₂)₂-O-, (H₃C-CH₂)-O-(CH₂)₂-O-(CH₂)₂-O- und (H₃C-CH₂)-O-(CH₂)₂-O-(CH₂)₂-O-(CH₂)₂-O-,
R⁵ für ein Wasserstoffatom steht
und
R⁶ für ein Wasserstoffatom steht
oder ein Stereoisomer, ein Tautomer, ein Hydrat, ein Solvat oder ein Salz davon oder ein Gemisch davon, oder
- einen Gd³⁺⁻-Komplex einer Verbindung der folgenden Formel (II): 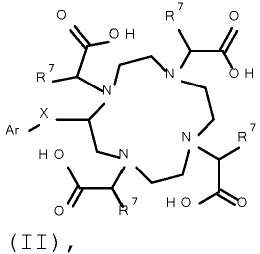 wobei
Ar für eine Gruppe steht, die ausgewählt ist aus 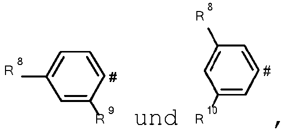
wobei # die Bindung an X ist,
X eine Gruppe ist, die ausgewählt ist aus CH₂, (CH₂)₂, (CH₂)₃, (CH₂)₄ und *-(CH₂)₂-O-CH₂-^{#}, wobei * die Bindung an Ar ist und ^{#} die Bindung an den Essigsäurerest ist,
R² für ein Wasserstoffatom oder eine Gruppe, die aus C₁-C₃-Alkyl, -CH₂OH, -(CH₂)₂OH und -CH₂OCH₃ ausgewählt ist, steht,
R⁸ für eine Gruppe steht, die ausgewählt ist aus C₂-C₄-Alkoxy, (H₃C-CH₂O)-(CH₂)₂-O-, (H₃C-CH₂O)-(CH₂)₂-O-(CH₂)₂-O-und (H₃C-CH₂O)-(CH₂)₂-O-(CH₂)₂-O-(CH₂)₂-O-,
R⁹ und R¹⁰ unabhängig voneinander ein Wasserstoffatom darstellen;
oder ein Stereoisomer, ein Tautomer, ein Hydrat, ein Solvat oder ein Salz davon oder ein Gemisch davon, oder das Kontrastmittel eine der folgenden Substanzen umfasst:
- Gadolinium(III)-2-[4,7,10-Tris(carboxymethyl)-1,4,7,10-tetrazacyclododec-1-yl]essigsäure,
- Gadolinium(III)-Ethoxybenzyldiethylentriaminpentaessigsäure,
- Gadolinium(III)-2-[3,9-Bis[1-carboxylato-4-(2,3-dihydroxypropylamino)-4-oxobutyl]-3,6,9,15-tetrazabicyclo[9.3.1]pentadeca-1(15),11,13-trien-6-yl]-5-(2,3-dihydroxypropylamino)-5-oxopentanoat,
- Dihydrogen-[(±)-4-Carboxy-5,8,11-tris(carboxymethyl)-1-phenyl-2-oxa-5,8,11-triazatridecan-13-oato(5-)]gadolinat(2-),
- Tetragadolinium-[4,10-Bis(carboxylatomethyl)-7-{3,6,12,15-tetraoxo-16-[4,7,10-tris(carboxylatomethyl)-1,4,7,10-tetraazacyclododecan-1-yl]-9,9-bis({[({2-[4,7,10-tris(carboxylatomethyl)-1,4,7,10-tetraazacyclododecan-1-yl]propanoyl}amino)acetyl]-amino}methyl)-4,7,11,14-tetraazahepta-decan-2-yl}-1,4,7,10-tetraazacyclododecan-1-yl]acetat,
- 2,2',2"-(10-{1-Carboxy-2-[2-(4-ethoxyphenyl)ethoxy]ethyl}-1,4,7,10-tetraazacyclododecan-1,4,7-triyl)triacetat,
- Gadolinium-2,2',2"-{10-[1-Carboxy-2-{4-[2-(2-ethoxyethoxy)ethoxy]phenyl}ethyl]-1,4,7,10-tetraazacyclododecan-1,4,7-triyl}triacetat,
- Gadolinium-2,2',2"-{10-[(1R)-1-Carboxy-2-{4-[2-(2-ethoxyethoxy)ethoxy]phenyl}ethyl]-1,4,7,10-tetraazacyclododecan-1,4,7-triyl}triacetat,
- Gadolinium-(2S,2'S,2"S)-2,2',2"-{10-[(1S)-1-Carboxy-4-{4-[2-(2-ethoxyethoxy)ethoxy]phenyl}butyl]-1,4,7,10-tetraazacyclododecan-1,4,7-triyl}tris(3-hydroxypropanoat),
- Gadolinium-2,2',2"-{10-[(1S)-4-(4-Butoxyphenyl)-1-carboxybutyl]-1,4,7,10-tetraazacyclododecan-1,4,7-triyl}triacetat,
- Gadolinium(III)-5,8-Bis(carboxylatomethyl)-2-[2-(methylamino)-2-oxoethyl]-10-oxo-2,5,8,11-tetraazadodecan-1-carboxylathydrat,
- Gadolinium(III)-2-[4-(2-Hydroxypropyl)-7,10-bis(2-oxido-2-oxoethyl)-1,4,7,10-tetrazacyclododec-1-yl]acetat,
- Gadolinium(III)-2,2',2"-(10-((2R,3S)-1,3,4-Trihydroxybutan-2-yl)-1,4,7,10-tetraazacyclododecan-1,4,7-triyl)triacetat,
- Gadolinium-2,2',2"-{(2S)-10-(carboxymethyl)-2-[4-(2-ethoxyethoxy)benzyl]-1,4,7,10-tetraazacyclododecan-1,4,7-triyl}triacetat,
- Gadolinium-2,2',2"-[10-(carboxymethyl)-2-(4-ethoxybenzyl)-1,4,7,10-tetraazacyclododecan-1,4,7-triyl]triacetat.

## Revendications

1. Procédé mis en œuvre par ordinateur d'entraînement d'un modèle d'apprentissage machine (Machine Learning Model, MLM) génératif, le procédé comprenant les faits :
- de fournir le modèle d'apprentissage machine génératif (MLM),
- de fournir des données d'entraînement (Training Data, TD), dans lequel les données d'entraînement (TD) comprennent, pour chaque objet de référence d'une pluralité d'objets de référence, une première représentation de référence (Reference Representation, RR1) représentant une zone d'examen de l'objet de référence sans agent de contraste, et une seconde représentation de référence (RR2) représentant la zone d'examen de l'objet de référence après application d'une première quantité d'un agent de contraste,
- d'entraîner le modèle d'apprentissage machine génératif (MLM), dans lequel le fait d'entraîner le modèle d'apprentissage machine génératif (MLM) comprend, pour chaque objet de référence de la pluralité d'objets de référence, les faits :
• d'amener le modèle d'apprentissage machine génératif (MLM) à générer une première représentation synthétique (Synthetic Representation, (SR1) de la zone d'examen de l'objet de référence sur la base de la première représentation de référence (RR1) et/ou de la seconde représentation de référence (RR2),
• de générer une deuxième représentation synthétique (SR2) de la zone d'examen de l'objet de référence, dans lequel le fait de générer la deuxième représentation synthétique (SR2) comprend : le fait de soustraire la première représentation de référence (RR1) de la première représentation synthétique (SR1),
• de générer une troisième représentation synthétique (SR3) de la zone d'examen de l'objet de référence, dans lequel le fait de générer la troisième représentation synthétique (SR3) comprend : le fait de réduire le contraste de la deuxième représentation synthétique (SR2),
• de générer une quatrième représentation synthétique (SR4) de la zone d'examen de l'objet de référence, dans lequel le fait de générer la quatrième représentation synthétique (SR4) comprend : le fait de soustraire la troisième représentation synthétique (SR3) de la première représentation synthétique (SR1) ou le fait d'additionner la troisième représentation synthétique (SR3) à la première représentation de référence (RR1),
• de déterminer des écarts entre la quatrième représentation synthétique (SR4) et la seconde représentation de référence (RR2),
• de réduire les écarts en modifiant des paramètres du modèle d'apprentissage machine génératif (MLM),
- de stocker le modèle d'apprentissage machine génératif entraîné (MLM^{t}) et/ou de transmettre le modèle d'apprentissage machine génératif entraîné (MLM^{t}) à un autre système d'ordinateur et/ou d'utiliser le modèle d'apprentissage machine génératif entraîné (MLM^{t}) pour générer une représentation synthétique (SR) de la zone d'examen d'un objet d'examen.

2. Procédé mis en œuvre par ordinateur de génération d'une représentation synthétique (SR) en utilisant un modèle d'apprentissage machine génératif entraîné (MLM^{t}), le procédé comprenant les faits :
- de fournir le modèle d'apprentissage machine génératif entraîné (MLM^{t}),
• dans lequel le modèle d'apprentissage machine génératif entraîné (MLM^{t}) a été entraîné sur des données d'entraînement (TD),
• dans lequel les données d'entraînement (TD) comprenaient, pour chaque objet de référence d'une pluralité d'objets de référence, une première représentation de référence (RR1) représentant une zone d'examen de l'objet de référence sans agent de contraste, et une seconde représentation de référence (RR2) représentant la zone d'examen de l'objet de référence après application d'une première quantité d'un agent de contraste,
• dans lequel le fait d'entraîner le modèle d'apprentissage machine génératif (MLM) comprenait, pour chaque objet de référence de la pluralité d'objets de référence, les faits :
∘ d'amener le modèle d'apprentissage machine génératif (MLM) à générer une première représentation synthétique (SR1) de la zone d'examen de l'objet de référence sur la base de la première représentation de référence (RR1) et/ou de la seconde représentation de référence (RR2),
∘ de générer une deuxième représentation synthétique (SR2) de la zone d'examen de l'objet de référence, dans lequel le fait de générer la deuxième représentation synthétique (SR2) comprend : le fait de soustraire la première représentation de référence (RR1) de la première représentation synthétique (SR1),
∘ de générer une troisième représentation synthétique (SR3) de la zone d'examen de l'objet de référence, dans lequel le fait de générer la troisième représentation synthétique (SR3) comprend : le fait de réduire le contraste de la deuxième représentation synthétique (SR2),
∘ de générer une quatrième représentation synthétique (SR4) de la zone d'examen de l'objet de référence, dans lequel le fait de générer la quatrième représentation synthétique (SR4) comprend : le fait de soustraire la troisième représentation synthétique (SR3) de la première représentation synthétique (SR1) ou d'additionner la troisième représentation synthétique (SR3) à la première représentation de référence (RR1),
∘ de déterminer des écarts entre la quatrième représentation synthétique (SR4) et la seconde représentation de référence (RR2),
∘ de réduire les écarts en modifiant des paramètres du modèle d'apprentissage machine génératif (MLM),
- de fournir une première représentation (R1) et/ou une seconde représentation (R2) de la zone d'examen d'un objet d'examen, dans lequel la première représentation (R1) représente la zone d'examen de l'objet d'examen sans agent de contraste, et la seconde représentation (R2) représente la zone d'examen de l'objet d'examen après application de la première quantité de the agent de contraste,
- d'amener le modèle d'apprentissage machine génératif entraîné (MLM^{t}) à générer une représentation synthétique (SR) de la zone d'examen de l'objet d'examen sur la base de la première représentation (R1) et/ou de la seconde représentation (R2),
- de sortir la représentation synthétique (SR) de la zone d'examen de l'objet d'examen et/ou de stocker la représentation synthétique (SR) de la zone d'examen de l'objet d'examen dans un composant de stockage de données et/ou de transmettre la représentation synthétique (SR) de la zone d'examen de l'objet d'examen à un système d'ordinateur distinct.

3. Procédé de la revendication 1 ou 2, dans lequel chaque objet de référence est un être vivant, par exemple un mammifère, par exemple un humain, et l'objet d'examen est un être vivant, par exemple un mammifère, par exemple un humain.

4. Procédé de l'une quelconque des revendications 1 à 3, dans lequel la zone d'examen est ou comprend un foie, rein, cœur, poumon, cerveau, estomac, une vessie, prostate, un intestin, sein, une thyroïde, un pancréas, utérus, ou une partie de ceux-ci, d'un mammifère, par exemple d'un humain.

5. Procédé de l'une quelconque des revendications 1 à 4, dans lequel chaque représentation est une représentation radiologique.

6. Procédé de l'une quelconque des revendications 1 à 5, dans lequel la représentation synthétique (SR) de la zone d'examen de l'objet d'examen représente la zone d'examen de l'objet d'examen après application d'une seconde quantité de l'agent de contraste, dans lequel la seconde quantité est supérieure à la première quantité.

7. Procédé de l'une quelconque des revendications 1 à 6, dans lequel le fait de réduire le contraste de la deuxième représentation synthétique (SR2) comprend : atténuation linéaire ou non linéaire de valeurs de gris ou de valeurs de couleur d'éléments d'image de la deuxième représentation synthétique (SR2).

8. Procédé de l'une quelconque des revendications 1 à 7, dans lequel le fait de réduire le contraste de la deuxième représentation synthétique (SR2) comprend : le fait de multiplier des valeurs de gris ou des valeurs de couleur d'éléments d'image de la deuxième représentation synthétique (SR2) par un facteur d'atténuation, dans lequel le facteur d'atténuation est supérieur à zéro et inférieur à 1.

9. Procédé de l'une quelconque des revendications 1 à 8, dans lequel le modèle d'apprentissage machine génératif (MLM) est ou comprend un ou plusieurs éléments parmi les suivants : réseau neuronal artificiel, réseau neuronal convolutif, auto-encodeur variationnel, réseau contradictoire génératif, transformateur, réseau de diffusion.

10. Procédé de l'une quelconque des revendications 1 à 10, dans lequel la zone d'examen est un foie humain ou comprend un foie humain ou fait partie d'un foie humain, et l'agent de contraste est un agent de contraste hépatobiliaire, par exemple un agent de contraste hépatobiliaire IRM.

11. Procédé de l'une quelconque des revendications 1 à 11, dans lequel la génération de la première représentation synthétique (SR1) de la zone d'examen de l'objet de référence est basée sur la première représentation de référence (RR1) et la seconde représentation de référence (RR2), et la génération de la représentation synthétique (SR) de la zone d'examen de l'objet d'examen est basée sur la première représentation (R1) et la seconde représentation (R2).

12. Système d'ordinateur (1), comprenant :
une unité de traitement (20) ou ; et
une mémoire (50) stockant un programme d'ordinateur (60) configuré pour réaliser, lorsqu'il est exécuté par l'unité de traitement (20) ou, une opération, l'opération comprenant les faits :
- de fournir le modèle d'apprentissage machine génératif entraîné (MLM^{t}),
• dans lequel le modèle d'apprentissage machine génératif entraîné (MLM^{t}) a été entraîné sur des données d'entraînement (TD),
• dans lequel les données d'entraînement (TD) comprenaient, pour chaque objet de référence d'une pluralité d'objets de référence, une première représentation de référence (RR1) représentant une zone d'examen de l'objet de référence sans agent de contraste, et une seconde représentation de référence (RR2) représentant la zone d'examen de l'objet de référence après application d'une première quantité d'un agent de contraste,
• dans lequel le fait d'entraîner le modèle d'apprentissage machine génératif (MLM) comprenait, pour chaque objet de référence de la pluralité d'objets de référence, les faits :
∘ d'amener le modèle d'apprentissage machine génératif (MLM) à générer une première représentation synthétique (SR1) de la zone d'examen de l'objet de référence sur la base de la première représentation de référence (RR1) et/ou de la seconde représentation de référence (RR2),
∘ de générer une deuxième représentation synthétique (SR2) de la zone d'examen de l'objet de référence, dans lequel le fait de générer la deuxième représentation synthétique (SR2) comprend : le fait de soustraire la première représentation de référence (RR1) de la première représentation synthétique (SR1),
∘ de générer une troisième représentation synthétique (SR3) de la zone d'examen de l'objet de référence, dans lequel le fait de générer la troisième représentation synthétique (SR3) comprend : le fait de réduire le contraste de la deuxième représentation synthétique (SR2),
∘ de générer une quatrième représentation synthétique (SR4) de la zone d'examen de l'objet de référence, dans lequel le fait de générer la quatrième représentation synthétique (SR4) comprend : le fait de soustraire la troisième représentation synthétique (SR3) de la première représentation synthétique (SR1) ou d'additionner la troisième représentation synthétique (SR3) à la première représentation de référence (RR1),
∘ de déterminer des écarts entre la quatrième représentation synthétique (SR4) et la seconde représentation de référence (RR2),
∘ de réduire les écarts en modifiant des paramètres du modèle d'apprentissage machine génératif (MLM),
- de fournir une première représentation (R1) et/ou une seconde représentation (R2) de la zone d'examen d'un objet d'examen, dans lequel la première représentation (R1) représente la zone d'examen de l'objet d'examen sans agent de contraste, et la seconde représentation (R2) représente la zone d'examen de l'objet d'examen après application de la première quantité de the agent de contraste,
- d'amener le modèle d'apprentissage machine génératif entraîné (MLM^{t}) à générer une représentation synthétique (SR) de la zone d'examen de l'objet d'examen sur la base de la première représentation (R1) et/ou de la seconde représentation (R2),
- de sortir la représentation synthétique (SR) de la zone d'examen de l'objet d'examen et/ou de stocker la représentation synthétique (SR) de la zone d'examen de l'objet d'examen dans un composant de stockage de données et/ou de transmettre la représentation synthétique (SR) de la zone d'examen de l'objet d'examen à un système d'ordinateur distinct.

13. Support de stockage non transitoire lisible par ordinateur, ayant, stocké sur celui-ci, un programme d'ordinateur qui, lorsqu'il est exécuté par une unité de traitement (20) d'un système d'ordinateur (1), amène le système d'ordinateur (1) à réaliser les étapes suivantes comprenant les faits :
- de fournir le modèle d'apprentissage machine génératif entraîné (MLM^{t}),
• dans lequel le modèle d'apprentissage machine génératif entraîné (MLM^{t}) a été entraîné sur des données d'entraînement (TD),
• dans lequel les données d'entraînement (TD) comprenaient, pour chaque objet de référence d'une pluralité d'objets de référence, une première représentation de référence (RR1) représentant une zone d'examen de l'objet de référence sans agent de contraste, et une seconde représentation de référence (RR2) représentant la zone d'examen de l'objet de référence après application d'une première quantité d'un agent de contraste,
• dans lequel le fait d'entraîner le modèle d'apprentissage machine génératif (MLM) comprenait, pour chaque objet de référence de la pluralité d'objets de référence, les faits :
∘ d'amener le modèle d'apprentissage machine génératif (MLM) à générer une première représentation synthétique (SR1) de la zone d'examen de l'objet de référence sur la base de la première représentation de référence (RR1) et/ou de la seconde représentation de référence (RR2),
∘ de générer une deuxième représentation synthétique (SR2) de la zone d'examen de l'objet de référence, dans lequel le fait de générer la deuxième représentation synthétique (SR2) comprend : le fait de soustraire la première représentation de référence (RR1) de la première représentation synthétique (SR1),
∘ de générer une troisième représentation synthétique (SR3) de la zone d'examen de l'objet de référence, dans lequel le fait de générer la troisième représentation synthétique (SR3) comprend : le fait de réduire le contraste de la deuxième représentation synthétique (SR2),
∘ de générer une quatrième représentation synthétique (SR4) de la zone d'examen de l'objet de référence, dans lequel le fait de générer la quatrième représentation synthétique (SR4) comprend : le fait de soustraire la troisième représentation synthétique (SR3) de la première représentation synthétique (SR1) ou d'additionner la troisième représentation synthétique (SR3) à la première représentation de référence (RR1),
∘ de déterminer des écarts entre la quatrième représentation synthétique (SR4) et la seconde représentation de référence (RR2),
∘ de réduire les écarts en modifiant des paramètres du modèle d'apprentissage machine génératif (MLM),
- de fournir une première représentation (R1) et/ou une seconde représentation (R2) de la zone d'examen d'un objet d'examen, dans lequel la première représentation (R1) représente la zone d'examen de l'objet d'examen sans agent de contraste, et la seconde représentation (R2) représente la zone d'examen de l'objet d'examen après application de la première quantité de the agent de contraste,
- d'amener le modèle d'apprentissage machine génératif entraîné (MLM^{t}) à générer une représentation synthétique (SR) de la zone d'examen de l'objet d'examen sur la base de la première représentation (R1) et/ou de la seconde représentation (R2),
- de sortir la représentation synthétique (SR) de la zone d'examen de l'objet d'examen et/ou de stocker la représentation synthétique (SR) de la zone d'examen de l'objet d'examen dans un composant de stockage de données et/ou de transmettre la représentation synthétique (SR) de la zone d'examen de l'objet d'examen à un système d'ordinateur distinct.

14. Utilisation d'un agent de contraste dans un examen d'une zone d'examen d'un objet d'examen, l'examen comprenant les faits :
- de fournir un modèle d'apprentissage machine génératif entraîné (MLM^{t}),
• dans lequel le modèle d'apprentissage machine génératif entraîné (MLMt) a été entraîné sur des données d'entraînement (TD),
• dans laquelle les données d'entraînement (TD) comprenaient, pour chaque objet de référence d'une pluralité d'objets de référence, une première représentation de référence (RR1) représentant une zone d'examen de l'objet de référence sans agent de contraste, et une seconde représentation de référence (RR2) représentant la zone d'examen de l'objet de référence après l'application d'une quantité de l'agent de contraste,
• dans lequel le fait d'entraîner le modèle d'apprentissage machine génératif (MLM) comprenait, pour chaque objet de référence de la pluralité d'objets de référence, les faits :
∘ d'amener le modèle d'apprentissage machine génératif (MLM) à générer une première représentation synthétique (SR1) de la zone d'examen de l'objet de référence sur la base de la première représentation de référence (RR1) et/ou de la seconde représentation de référence (RR2),
∘ de générer une deuxième représentation synthétique (SR2) de la zone d'examen de l'objet de référence, dans lequel le fait de générer la deuxième représentation synthétique (SR2) comprend : le fait de soustraire la première représentation de référence (RR1) de la première représentation synthétique (SR1),
∘ de générer une troisième représentation synthétique (SR3) de la zone d'examen de l'objet de référence, dans lequel le fait de générer la troisième représentation synthétique (SR3) comprend : le fait de réduire le contraste de la deuxième représentation synthétique (SR2),
∘ de générer une quatrième représentation synthétique (SR4) de la zone d'examen de l'objet de référence, dans lequel le fait de générer la quatrième représentation synthétique (SR4) comprend : le fait de soustraire la troisième représentation synthétique (SR3) de la première représentation synthétique (SR1) ou d'additionner la troisième représentation synthétique (SR3) à la première représentation de référence (RR1),
∘ de déterminer des écarts entre la quatrième représentation synthétique (SR4) et la seconde représentation de référence (RR2),
∘ de réduire les écarts en modifiant des paramètres du modèle d'apprentissage machine génératif (MLM),
- de générer une première représentation (R1) et/ou une seconde représentation (R2) de la zone d'examen d'un objet d'examen, dans laquelle la première représentation (R1) représente la zone d'examen de l'objet d'examen sans agent de contraste, et la seconde représentation (R2) représente la zone d'examen de l'objet d'examen après application de la quantité de l'agent de contraste,
- d'amener le modèle d'apprentissage machine génératif entraîné (MLM^{t}) à générer une représentation synthétique (SR) de la zone d'examen de l'objet d'examen sur la base de la première représentation (R1) et/ou de la seconde représentation (R2),
- de sortir la représentation synthétique (SR) de la zone d'examen de l'objet d'examen et/ou de stocker la représentation synthétique (SR) de la zone d'examen de l'objet d'examen dans un composant de stockage de données et/ou de transmettre la représentation synthétique (SR) de la zone d'examen de l'objet d'examen à un système d'ordinateur distinct.

15. Utilisation de l'agent de contraste de la revendication 14, dans laquelle l'agent de contraste comprend
- un complexe Gd³⁺ d'un composé de la formule (I) 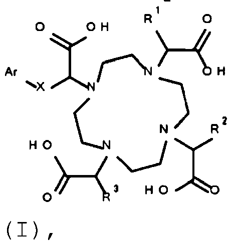 où
Ar est un groupe sélectionné parmi 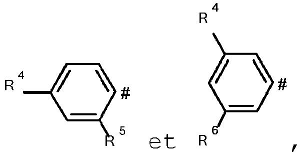
où # est la liaison à X,
X est un groupe sélectionné parmi CH₂, (CH₂)₂, (CH₂)₃, (CH₂)₄, et *-(CH₂)₂-O-CH₂-^{#},
où * est la liaison à Ar ^{#} est la liaison au résidu d'acide acétique,
R¹, R², et R³ rsont chacun indépendamment un atome d'hydrogène ou un groupe sélectionné parmi alkyle en C₁-C₃, -CH₂OH, -(CH₂)₂OH, et -CH₂OCH₃,
R⁴ est un groupe sélectionné parmi alkoxy en C₂-C₄, (H₃C-CH₂)-O-(CH₂)₂-O-, (H₃C-CH₂)-O-(CH₂)₂-O-(CH₂)₂-O-, et (H₃C-CH₂)-O-(CH₂)₂-O-(CH₂)₂-O-(CH₂)₂-O-,
R⁵ est un atome d'hydrogène,
et
R⁶ est un atome d'hydrogène,
ou un stéréoisomère, tautomère, hydrate, solvate ou sel de ceux-ci, ou un mélange de ceux-ci, ou
- un complexe Gd³⁺ d'un composé de la formule (II), 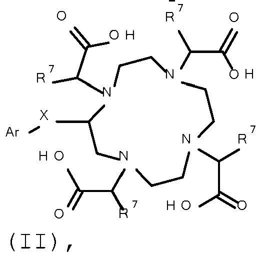 où
Ar est un groupe sélectionné parmi 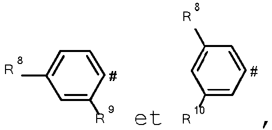
où # est la liaison à X,
X est un groupe sélectionné parmi CH₂, (CH₂)₂, (CH₂)₃, (CH₂)₄, et *-(CH₂)₂-O-CH₂-^{#}, où * est la liaison à Ar et Ar et ^{#} est la liaison au résidu d'acide acétique,
R⁷ représente un atome d'hydrogène ou un groupe choisi parmi un alkyle en C₁-C₃, -CH₂OH, -(CH₂)₂OH et -CH₂OCH₃ ; R⁸ représente un groupe choisi parmi un alcoxy en C₂-C₄, (H₃C-CH₂O)-(CH₂)₂-O-, (H₃C-CH₂O)-(CH₂)₂-O-(CH₂)₂-O- et (H₃C-CH₂O)-(CH₂)₂-O-(CH₂)₂-O-(CH₂)₂-O- ;
R⁹ et R¹⁰ représentent indépendamment un atome d'hydrogène ;
ou un stéréoisomère, tautomère, hydrate, solvate ou sel de ceux-ci, ou un mélange de ceux-ci, ou
l'agent de contraste comprend une des substances suivantes :
- acide 2-[4,7,10-tris(carboxyméthyl)-1,4,7,10-tétrazacyclododéc-1-yl]acétique de gadolinium(III),
- acide éthoxybenzyldiéthylènetriaminepentaacétique de gadolinium(III),
- 2-[3,9-bis[1-carboxylato-4-(2,3-dihydroxypropylamino)-4-oxobutyl]-3,6,9,15-tétrazabicyclo[9.3.1]pentadéca-1(15),11,13-trien-6-yl]-5-(2,3-dihydroxypropylamino)-5-oxopentanoate de gadolinium(III),
- [(±)-4-carboxy-5,8,11-tris(carboxyméthyl)-1-phényl-2-oxa-5,8,11-triazatridécan-13-oato(5-)]gadolinate(2-) de dihydrogène,
- [4,10-bis(carboxylatométhyl)-7-{3,6,12,15-tétraoxo-16-[4,7,10-tris(carboxylatométhyl)-1,4,7,10-tétraazacyclododécan-1-yl]-9,9-bis({[({2-[4,7,10-tris(carboxylatométhyl)-1,4,7,10-tétraazacyclododécan-1-yl]propanoyl}amino)acetyl]-amino}méthyl)-4,7,11,14-tétraazahepta-décan-2-yl}-1,4,7,10-tétraazacyclododécan-1-yl]acétate de tétragadolinium,
- 2,2',2"-(10-{1-carboxy-2-[2-(4-éthoxyphényl)éthoxy]éthyl}-1,4,7,10-tétraazacyclododécane-1,4,7-triyl)triacétate,
- 2,2',2''-{10-[1-carboxy-2-{4-[2-(2-éthoxyéthoxy)éthoxy]phényl}éthyl]-1,4,7,10-tétraazacyclododécane-1,4,7-triyl}triacétate de gadolinium,
- 2,2',2"-{10-[(1R)-1-carboxy-2-{4-[2-(2-éthoxyéthoxy)éthoxy]phényl}éthyl]-1,4,7,10-tétraazacyclododécane-1,4,7-triyl}triacétate de gadolinium,
- (2S,2'S,2"S)-2,2',2"-{10-[(1S)-1-carboxy-4-{4-[2-(2-éthoxyéthoxy)éthoxy]phényl}butyl]-1,4,7,10-tétraazacyclododécane-1,4,7-triyl}tris(3-hydroxypropanoate) de gadolinium,
- 2,2',2"-{10-[(1S)-4-(4-butoxyphényl)-1-carboxybutyl]-1,4,7,10-tétraazacyclododécane-1,4,7-triyl}triacétate de gadolinium,
- hydrate de 5,8-bis(carboxylatométhyl)-2-[2-(méthylamino)-2-oxoéthyl]-10-oxo-2,5,8,11-tétraazadodécane-1-carboxylate de gadolinium(III),
- 2-[4-(2-hydroxypropyl)-7,10-bis(2-oxido-2-oxoéthyl)-1,4,7,10-tétrazacyclododéc-1-yl]acétate de gadolinium(III),
- 2,2',2''-(10-((2R,3S)-1,3,4-trihydroxybutan-2-yl)-1,4,7,10-tétraazacyclododécane-1,4,7-triyl)triacétate de gadolinium(III),
- gadolinium-2,2',2"-{(2S)-10-(carboxyméthyl)-2-[4-(2-éthoxyéthoxy)benzyl]-1,4,7,10-tétraazacyclododécane-1,4,7-triyl}triacétate,
- gadolinium-2,2',2"-[10-(carboxyméthyl)-2-(4-éthoxybenzyl)-1,4,7,10-tétraazacyclododécane-1,4,7-triyl]triacétate.

16. Kit comprenant un programme d'ordinateur selon la revendication 13 et un agent de contraste, dans lequel l'agent de contraste comprend
- un complexe Gd³⁺ d'un composé de la formule (I), 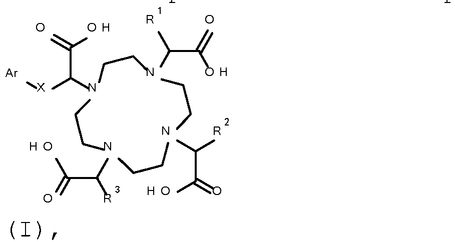 où
Ar est un groupe sélectionné parmi 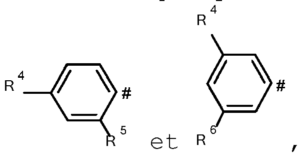
où # est la liaison à X,
X est un groupe sélectionné parmi CH₂, (CH₂)₂, (CH₂)₃, (CH₂)₄, et *-(CH₂)₂-O-CH₂-^{#},
où * est la liaison à Ar ^{#} est la liaison au résidu d'acide acétique,
R¹, R², et R³ rsont chacun indépendamment un atome d'hydrogène ou un groupe sélectionné parmi alkyle en C₁-C₃, -CH₂OH, -(CH₂)₂OH, et -CH₂OCH₃,
R⁴ est un groupe sélectionné parmi alkoxy en C₂-C₄, (H₃C-CH₂)-O-(CH₂)₂-O-, (H₃C-CH₂)-O-(CH₂)₂-O-(CH₂)₂-O-, et (H₃C-CH₂)-O-(CH₂)₂-O-(CH₂)₂-O-(CH₂)₂-O-,
R⁵ est un atome d'hydrogène,
et
R⁶ est un atome d'hydrogène,
ou un stéréoisomère, tautomère, hydrate, solvate ou sel de ceux-ci, ou un mélange de ceux-ci, ou
- un complexe Gd³⁺ d'un composé de la formule (II), 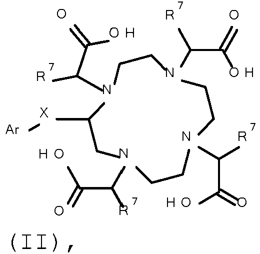 où
Ar est un groupe sélectionné parmi 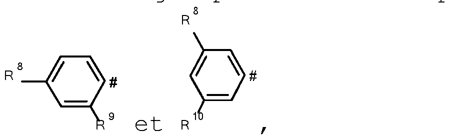
où # est la liaison à X,
X est un groupe sélectionné parmi CH₂, (CH₂)₂, (CH₂)₃, (CH₂)₄, et *-(CH₂)₂-O-CH₂-^{#}, où * est la liaison à Ar et Ar et ^{#} est la liaison au résidu d'acide acétique,
R⁷ représente un atome d'hydrogène ou un groupe choisi parmi un alkyle en C₁-C₃, -CH₂OH, -(CH₂)₂OH et -CH₂OCH₃ ; R⁸ représente un groupe choisi parmi un alcoxy en C₂-C₄, (H₃C-CH₂O)-(CH₂)₂-O-, (H₃C-CH₂O)-(CH₂)₂-O-(CH₂)₂-O- et (H₃C-CH₂O)-(CH₂)₂-O-(CH₂)₂-O-(CH₂)₂-O- ;
R⁹ et R¹⁰ représentent indépendamment un atome d'hydrogène ;
ou un stéréoisomère, tautomère, hydrate, solvate ou sel de ceux-ci, ou un mélange de ceux-ci, ou
l'agent de contraste comprend une des substances suivantes :
- acide 2-[4,7,10-tris(carboxyméthyl)-1,4,7,10-tétrazacyclododéc-1-yl]acétique de gadolinium(III),
- acide éthoxybenzyldiéthylènetriaminepentaacétique de gadolinium(III),
- 2-[3,9-bis[1-carboxylato-4-(2,3-dihydroxypropylamino)-4-oxobutyl]-3,6,9,15-tétrazabicyclo[9.3.1]pentadéca-1(15),11,13-trien-6-yl]-5-(2,3-dihydroxypropylamino)-5-oxopentanoate de gadolinium(III),
- [(±)-4-carboxy-5,8,11-tris(carboxyméthyl)-1-phényl-2-oxa-5,8,11-triazatridécan-13-oato(5-)]gadolinate(2-) de dihydrogène,
- [4,10-bis(carboxylatométhyl)-7-{3,6,12,15-tétraoxo-16-[4,7,10-tris(carboxylatométhyl)-1,4,7,10-tétraazacyclododécan-1-yl]-9,9-bis({[({2-[4,7,10-tris(carboxylatométhyl)-1,4,7,10-tétraazacyclododécan-1-yl]propanoyl}amino)acetyl]-amino}méthyl)-4,7,11,14-tétraazahepta-décan-2-yl}-1,4,7,10-tétraazacyclododécan-1-yl]acétate de tétragadolinium,
- 2,2',2"-(10-{1-carboxy-2-[2-(4-éthoxyphényl)éthoxy]éthyl}-1,4,7,10-tétraazacyclododécane-1,4,7-triyl)triacétate,
- 2,2',2''-{10-[1-carboxy-2-{4-[2-(2-éthoxyéthoxy)éthoxy]phényl}éthyl]-1,4,7,10-tétraazacyclododécane-1,4,7-triyl}triacétate de gadolinium,
- 2,2',2''-{10-[(1R)-1-carboxy-2-{4-[2-(2-éthoxyéthoxy)éthoxy]phényl}éthyl]-1,4,7,10-tétraazacyclododécane-1,4,7-triyl}triacétate de gadolinium,
- (2S,2'S,2"S)-2,2',2"-{10-[(1S)-1-carboxy-4-{4-[2-(2-éthoxyéthoxy)éthoxy]phényl}butyl]-1,4,7,10-tétraazacyclododécane-1,4,7-triyl}tris(3-hydroxypropanoate) de gadolinium,
- 2,2',2"-{10-[(1S)-4-(4-butoxyphényl)-1-carboxybutyl]-1,4,7,10-tétraazacyclododécane-1,4,7-triyl}triacétate de gadolinium,
- hydrate de 5,8-bis(carboxylatométhyl)-2-[2-(méthylamino)-2-oxoéthyl]-10-oxo-2,5,8,11-tétraazadodécane-1-carboxylate de gadolinium(III),
- 2-[4-(2-hydroxypropyl)-7,10-bis(2-oxido-2-oxoéthyl)-1,4,7,10-tétrazacyclododéc-1-yl]acétate de gadolinium(III),
- 2,2',2"-(10-((2R,3S)-1,3,4-trihydroxybutan-2-yl)-1,4,7,10-tétraazacyclododécane-1,4,7-triyl)triacétate de gadolinium(III),
- gadolinium-2,2',2"-{(2S)-10-(carboxyméthyl)-2-[4-(2-éthoxyéthoxy)benzyl]-1,4,7,10-tétraazacyclododécane-1,4,7-triyl}triacétate,
- gadolinium-2,2',2"-[10-(carboxyméthyl)-2-(4-éthoxybenzyl)-1,4,7,10-tétraazacyclododécane-1,4,7-triyl]triacétate.
